# EUROPÄISCHE PATENTSCHRIFT

(11) **EP 2 910 236 B1**
(45) Veröffentlichungstag und Bekanntmachung des Hinweises auf die Patenterteilung: **12.02.2020**
(21) Anmeldenummer: 14156436.9
(22) Anmeldetag: 24.02.2014
(51) Int. Cl.: A61K 6/087, C07D 251/34, C08L 75/04, C08L 81/02

(54) **Dentalmaterialien auf der Basis von geruchsarmen Thiolen**
Dental materials based on low-odour thiols
Matériaux dentaires à base de thiols à faible odeur

(43) Veröffentlichungstag der Anmeldung: 26.08.2015
(73) Patentinhaber: Ivoclar Vivadent AG, 9494 Schaan (LI)
(72) Erfinder: Moszner, Norbert, 9493 Mauren (LI); Burtscher, Peter, A-6830 Rankweil (AT); Fischer, Urs-Karl, 9320 Arbon (CH); Ritter, Helmut, 42111 Wuppertal (DE); Tabatabai, Monir, 40225 Düsseldorf (DE); Utterodt, Andreas, 61267 Neu-Anspach (DE); Reinelt, Sebastian, 40215 Dusseldorf (DE)
(74) Vertreter: Uexküll & Stolberg

(56) Entgegenhaltungen:
- WO-A2-2005/086911

## Beschreibung

Die vorliegende Erfindung betrifft thermisch- und/oder lichthärtende Dentalmaterialien zur Herstellung von dentalen Zementen und Füllungskompositen und für Inlay, Onlays, Kronen, Brücken oder Verblendmaterialien.

Dentale Komposite enthalten gewöhnlich eine polymerisierbare organische Matrix und einen oder mehrere Füllstoffe. Als polymerisierbare organische Matrix wird in den meisten Fällen eine Mischung von Monomeren, Initiator-Komponenten, Stabilisatoren und Pigmenten verwendet, wobei als Harze oft Mischungen von Dimethacrylaten eingesetzt werden. Die Aushärtung derartiger Materialien kann durch thermische, redoxinitiierte oder lichtinduzierte radikalische Polymerisation erfolgen.

Ein wesentlicher Nachteil der radikalischen Polymerisation von dentalen Kompositen ist die durch den Polymerisationsschrumpf (ΔV_{P}) der eingesetzten Methacrylatmonomere bedingte Volumenkontraktion, die zur einer sehr nachteiligen Randspaltbildung bei Füllungskompositen führen kann. Der Polymerisationsschrumpf hängt linear von der Konzentration der Doppelbindungen im Volumen ab und nimmt dementsprechend mit zunehmendem Molekulargewicht der Monomere in der Harzmischung oder steigendem Volumenanteil an Füllstoffen im Komposit ab. Ausserdem steigt der Polymerisationsschrumpf mit wachsender Funktionalität der Monomeren und höherem Doppelbindungsumsatz der Polymerisation.

Bei der Polymerisation von monofunktionellen Methacrylaten, wie z.B. MMA (ΔV_{P} = 21,0 Vol.-%), führt die Polymerisationsschrumpfung nicht zum Aufbau einer Polymerisationskontraktionsspannung (PKS), weil die Verringerung des Volumens durch Fliessen der gebildeten Makromoleküle kompensiert werden kann. Im Falle der vernetzenden Polymerisation von multifunktionellen Methacrylaten kommt es aber am sogenannten Gelpunkt schon innerhalb von wenigen Sekunden, d.h. schon bei geringem Monomerumsatz zur Bildung eines dreidimensionalen polymeren Netzwerkes, so dass der Polymerisationsschrumpf nicht mehr durch visviskoses Fliessen kompensiert werden kann und sich im Material mit zunehmenden Monomerumsatz eine erhebliche PKS aufbaut.

Im Vergleich zur vernetzenden radikalischen Polymerisation, die nach einem Kettenwachstumsmechanismus abläuft, zeigen vernetzende Polyreaktionen mit Stufenwachstumsmechanismus eine Gelbildung erst bei deutlich höherem Monomerumsatz. Dabei kann der Monomerumsatz am Gelpunkt durch die Funktionalität der Monomerbausteine und das anfängliche Stoffmengenverhältnis beeinflusst werden (vgl. H.-G. Elias, Makromoleküle , Bd. 1, 6. Aufl., Weinheim etc. 1999, 478-480).

Es ist bekannt, dass sich die vernetzende Thiol-En-Polyaddition durch einen nahezu vollständigen Doppelbindungsumsatz und einen deutlich geringeren Polymerisationsschrumpf im Vergleich zur radikalischen Polymerisation von multifunktionellen Methacrylaten auszeichnet. So beträgt die Volumenkontraktion pro polymerisierter (Meth)acrylat-Doppelbindung ca. 22-23 cm³/mol, während bei der Thiol-En-Reaktion die Volumenkontraktion nur bei 12-15 cm³ pro mol umgesetzter Doppelbindung liegt (vgl. M. Patel, M. Braden, K. W. M. Davy, Biomaterials 8 (1987) 53-56). Ausserdem verlaufen vernetzende Thiol-En-Polyadditionen nach einem Stufenwachstumsmechanismus und zeigen deshalb eine im Vergleich zur Dimethacrylatpolymerisation signifikant verlängerte Prägelphase, was zusätzlich zur Verringerung der PKS führt. Weiterhin zeichnen sich Thiol-En-Systeme durch eine geringe Sauerstoffinhibierung aus. Nachteilig an Thiol-En-Systemen sind ihre im Vergleich zu Methacrylaten verschlechterten mechanischen Eigenschaften nach der Härtung.

Cramer et al., Dent. Mater. 26 (2010), 21-28, berichten, dass durch die Kombination von Thiol-Norbonen- und Thiol-Allylether-Systemen mit den Dimethacrylaten BisGMA und TEGDMA Materialien erhalten werden können, die hinsichtlich des Elastizitätsmoduls und der Biegefestigkeit mit BisGMA/TEGDMA-Systemen vergleichbar sind, die aber im Vergleich zu BisGMA/TEGDMA eine deutlich verringerte PKS aufweisen. Die verwendeten Thiole sollen sich durch eine verbesserte Lagerstabilität und einen geringen Geruch auszeichnen.

Gemäß Cramer et al., Dent. Mater. 26 (2010) 799-806, soll ein stöchiometrisches Verhältnis von Thiol zu En von 3:1 in ternären Methacrylat-Thiol-En-Systemen vorteilhaft gegenüber einem Verhältnis von 1:1 sein.

US 2012/0256338 A1, US 8,192,673 B2, US 7,838,571 B2 und US 2009/0270528 A1 offenbaren lichthärtende dentale Restaurationsmaterialien, die neben Methacrylaten mindestens 10 Gew.-% einer Mischung aus Thiol-Monomer und En-Monomer enthalten. Ein bevorzugtes Thiol ist Pentaerythritoltetramercaptopropionat (PETMP).

Die WO 2005/086911 A2 offenbart polymerisierbare Dentalwerkstoffe, die eine oder mehrere Polythiolverbindungen und eine oder mehrere Polyvinylverbindungen enthalten, wobei eine oder beide Verbindungen Oligomere sind. Die Zusammensetzungen können zusätzlich einen oder mehrere Füllstoffe oder Photoinitiatoren enthalten. Sie sollen sich nach der Härtung durch eine geringe Schrumpfung und einen verringerten Geruch auszeichnen.

Trotz der genannten Vorteile haben Thiol-En-Harze bisher keine praktische Anwendung in dentalen Kompositen gefunden, da vor allem der Geruch der Thiol-Komponente für eine dentale Anwendung nicht akzeptabel ist. Zudem ist die Lagerstabilität herkömmlicher Thiole unzureichend. Viele Thiole lassen sich zwar mit geeigneten Vorrichtungen wie z.B. einem Fallfilm- oder Dünnschichtverdampfer nahezu geruchsfrei herstellen, nehmen jedoch bei der Lagerung durch Zersetzung wieder einen intensiven Mercaptangeruch an.

Der Erfindung liegt die Aufgabe zugrunde, Dentalwerkstoffe bereitzustellen, die sich im Vergleich zu auf Dimethacrylaten basierenden Materialien durch einen geringeren Restmonomergehalt und eine verringerte Polymerisationsspannung bei vergleichbaren mechanischen Eigenschaften auszeichnen. Zudem sollen sie über für dentale Zwecke geeignete olfaktorische Ei-Eigenschaften und eine hohe Lagerstabilität verfügen und auch nach längerer Lagerung keinen unangenehmen Geruch annehmen. Außerdem sollen sie eine für die dentale Anwendung geeignete Hydrolysestabilität und einen geringen Polymerisationsschrumpf aufweisen.

Die Aufgabe wird erfindungsgemäss durch Dentalwerkstoffe gelöst, die mindestens eine En-Verbindung mit zwei oder mehr C-C-Mehrfachbindungen, eine Monoacyltrialkyl- oder Diacyldialkylgermanium-Verbindung als Photoinitiator für die radikalische Polymerisation und mindestens ein Thiol gemäß der allgemeinen Formel (I) enthalten, 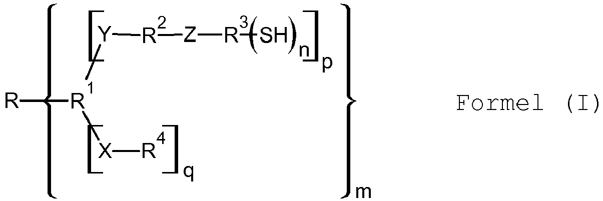 in der
- R: -SO₂-, ein linearer oder verzweigter aliphatischer C₁₋₂₀-Rest, ein aromatischer C₆₋₂₀-Rest, ein cycloaliphatischer C₃₋₁₈-Rest oder ein heterocyclischer Rest mit 3 bis 17 C-Atomen und 1 bis 3 Heteroatomen ist, die aus N, 0 und S ausgewählt sind;
- R¹: entfällt oder ein linearer oder verzweigter aliphatischer C₁₋₁₂-Rest, ein aromatischer C₆₋₂₀-Rest, ein cycloaliphatischer C₃₋₁₈-Rest oder ein heterocyclischer Rest mit 3 bis 17 C-Atomen und 1 bis 3 Heteroatomen ist, die aus N, 0 und S ausgewählt sind;
- R²: entfällt oder ein linearer oder verzweigter aliphatischer C₁₋₂₀-Rest, der durch 0 oder S unterbrochen sein kann, ein aromatischer C₆₋₁₀-Rest, der durch CH₃, CH₂CH₃, OH, OCH₃ oder -O-CO-CH₃ substituiert sein kann;
- R³: entfällt oder ein linearer oder verzweigter aliphatischer C₁₋₂₀-Rest, der durch 0 oder S unterbrochen sein kann, ein aromatischer C₆₋₁₀-Rest, der durch CH₃, CH₂CH₃, OH, OCH₃ oder -O-CO-CH₃ substituiert sein kann;
- R⁴: ein C₁₋₆-Alkylrest ist;
- X, Y: unabhängig voneinander O, S, CO-NH, O-CO-NH oder NH-CO-NH sind oder entfallen;
- Z: O, S, CO-NH, O-CO-NH oder NH-CO-NH ist oder entfällt;
- m: eine ganze Zahl von 1 bis 4 ist;
- n: eine ganze Zahl von 1 bis 4 ist;
- p: eine ganze Zahl von 1 bis 6 ist;
- q: eine ganze Zahl von 0 bis 4 ist,
wobei n, p und m so gewählt werden, dass das Thiol insgesamt mindestens 3, vorzugsweise 4 bis 6 SH-Gruppen aufweist.

Die obige Formel ist so zu verstehen, dass die in Klammern stehenden Ausdrücke dann, wenn m, p bzw. q größer als 1 sind, jeweils gleich oder verschieden sein können. Beispielsweise kann -[Y-R²-Z-R³-(SH)ₙ]₂ für zwei identische Gruppen -[Y-R²-Z-R³-(SH)ₙ] oder für zwei unterschiedliche Gruppen -[Y-R²-Z-R³-(SH)ₙ] und -[Y'-R²'-Z'-R³'-(SH)_{n'}] stehen. Bevorzugt sind Verbindungen, in denen die Klammerausdrücke jeweils identische Bedeutungen haben.

Die Formel erstreckt sich nur auf solche Verbindungen, die mit der chemischen Valenzlehre vereinbar sind. Beispielsweise kann dann, wenn R¹ für ein Kohlenstoffatom steht, die Summe von p und q nicht größer als drei sein, und m muss eins sein, wenn R ein Wasserstoffatom ist. Wenn R¹ entfällt, sind die in Klammern stehenden Ausdrücke direkt mit R verbunden. Wenn R -SO₂- ist, muss dann, wenn R¹ nicht entfällt, m = 2 sein, und wenn R¹ entfällt, muss die Summe von p und q = 2 sein. Wenn R² entfällt, dann entfällt vorzugsweise auch Y, und wenn R³ entfällt, entfällt vorzugsweise auch Z.

Vorzugsweise haben die Variablen der Formel (I) die folgenden Bedeutungen:
- R: -SO₂-, ein linearer oder verzweigter aliphatischer C₁₋₁₂-Rest, ein aromatischer C₆₋₁₈-Rest, ein cycloaliphatischer C₅₋₈-Rest oder ein heterocyclischer Rest mit 3 bis 5 C-Atomen, 1 bis 3 Heteroatomen und insgesamt 5-8 Ringatomen, wobei die Heteroatome aus N, O und S ausgewählt sind;
- R¹: entfällt, ein linearer oder verzweigter aliphatischer C₁₋₁₀-Rest oder ein aromatischer C₆₋₁₀-Rest;
- R²: entfällt oder ein linearer oder verzweigter aliphatischer C₁₋₁₀-Rest oder ein Phenylrest;
- R³: ein linearer oder verzweigter aliphatischer C₁₋₁₀-Rest oder ein Phenylrest;
- R⁴: ein C₁₋₄-Alkylrest;
- X: O oder entfällt;
- Y: O oder entfällt;
- Z: O oder entfällt;
- m: 1, 2 oder 3;
- n: 1 oder 2;
- p: 1, 2 oder 3;
- q: 0, 1 oder 2.

Ganz besonders bevorzugt sind Thiole der Formel (I), in der die Variablen die folgenden Bedeutungen haben:
- R: -SO₂-, ein linearer oder verzweigter aliphatischer C₁₋₆-Rest, ein aromatischer C₆-Rest, ein cycloaliphatischer C₅₋₈-Rest oder ein 1,3,5-Triazin-2,4,6-trion-rest;
- R¹: entfällt, ein linearer oder verzweigter aliphatischer C₁₋₆-Rest oder ein Phenylrest;
- R²: entfällt oder ein linearer oder verzweigter aliphatischer C₁₋₃-Rest ;
- R³: ein linearer oder verzweigter aliphatischer C₂₋₆-Rest;
- R⁴: ein C₁₋₃-Alkylrest;
- X: O oder entfällt;
- Y: O oder entfällt;
- Z: O oder entfällt;
- m: 2 oder 3;
- n: 1;
- p: 1 oder 2;
- q: 0 oder 1.

Niedermolekulare tri- oder höherfunktionalisierte Mercaptoverbindungen der Formel (I) sind teilweise bekannt und lassen sich nach bekannten Synthesemethoden einfach herstellen. Beispielsweise sind tri- oder höherfunktionalisierte Mercaptoverbindungen aus entsprechenden tri- oder höherfunktionalisierten Allylverbindungen durch Addition von Thioessigsäure und nachfolgende Esterspaltung gemäß literaturbekannten Methoden zugänglich (vgl. S. A. Svarovsky, Z. Szekely, J. J. Barchi, Tetrahedron: Asymmetry 16 (2005) 587-598; WO 98/58294 A1; US 4,266,055).

### Allgemeines Beispiel (n = 1, R³ = n-Propyl):

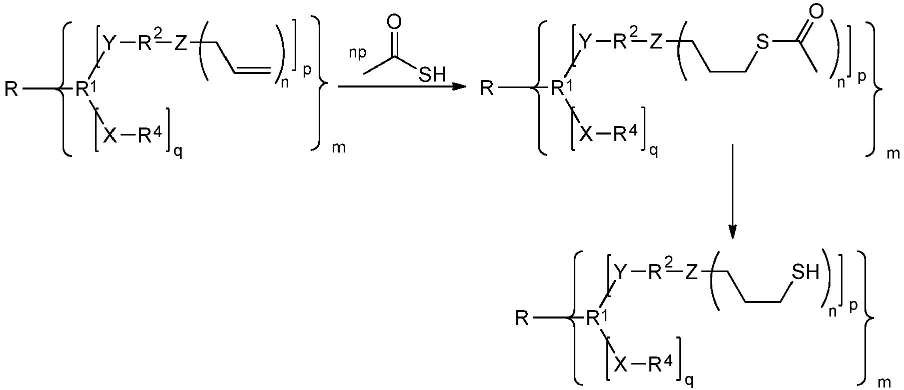

Ein konkretes Beispiel ist:

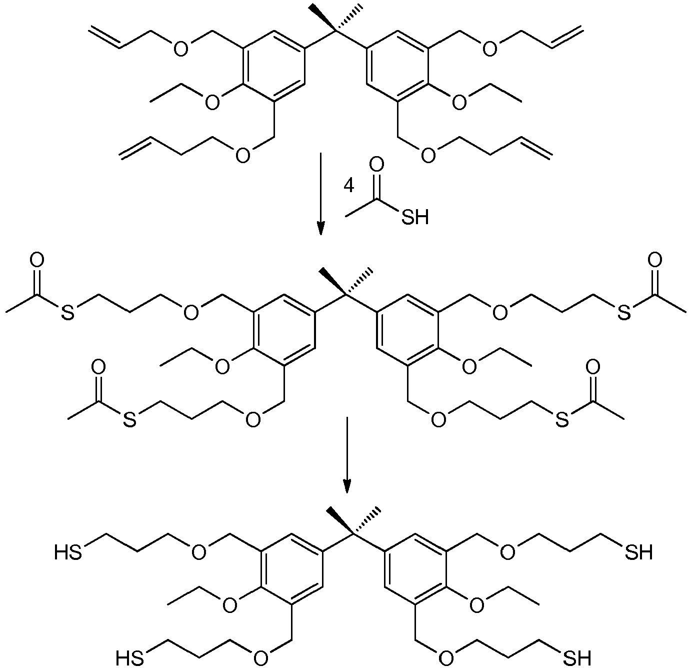

Geht man dabei von Propargylderivaten aus, so können gleichzeitig 2 Mercaptogruppen pro C-C-Mehrfachbindung eingeführt werden.

### Allgemeines Beispiel (n = 2, R³ = Iso-Propylen):

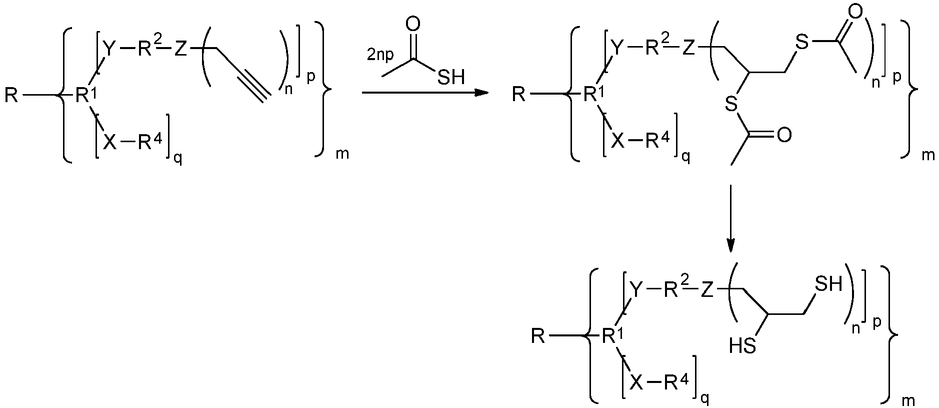

Ein konkretes Beispiel ist:

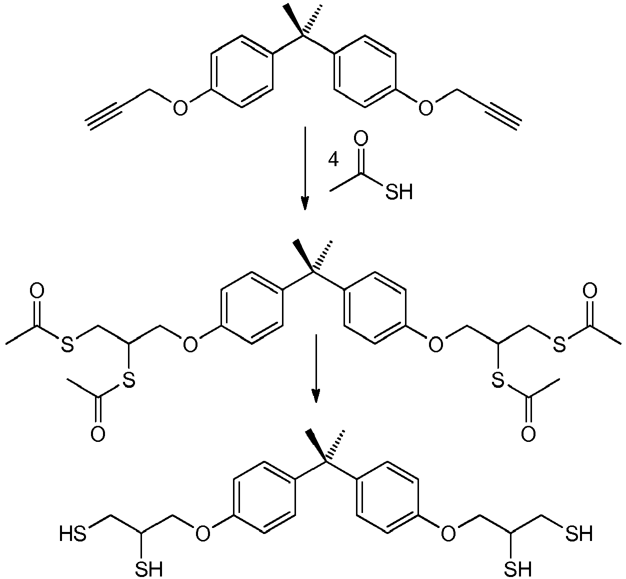

Niedermolekulare tri- oder höherfunktionalisierte Mercaptoverbindungen der Formel I lassen sich weiterhin durch nucleophile Substitution mit Schwefelnukleophilen wie Thioharnstoff etc. aus entsprechenden tri- oder höherfunktionalisierten Bromalkanen und nachfolgender Hydrolyse gemäß literaturbekannter Methoden herstellen (vgl. A. W. Snow, E. E. Foos, Synthesis 4 (2003) 509-512; J. Houk, G. M. Whitesides, J. Am. Chem. Soc. 109 (1987) 6825-6836).

### Allgemeines Beispiel (n = 2, R³ = Methylen):

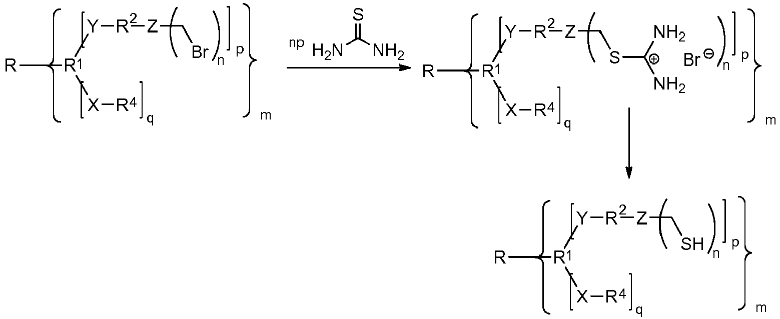

Ein konkretes Beispiel ist:

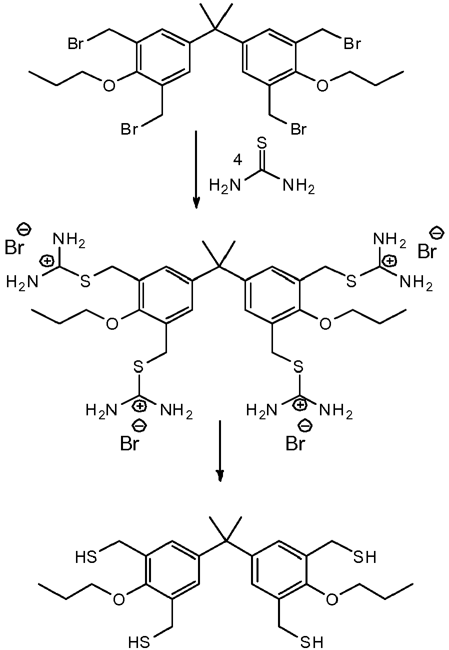

Bevorzugte Beispiele für die erfindungsgemässen tri- oder höherfunktionalisierte Mercaptoverbindungen der allgemeinen Formel I sind:

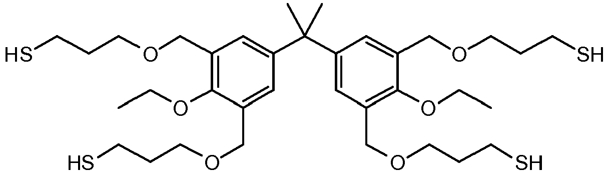

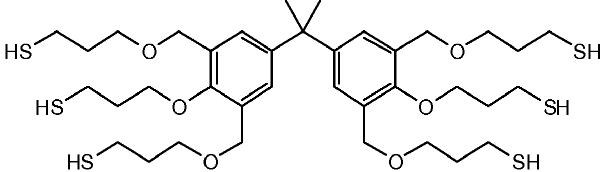

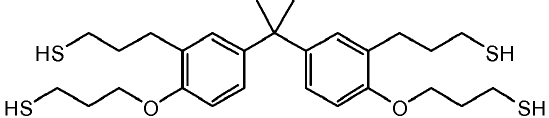

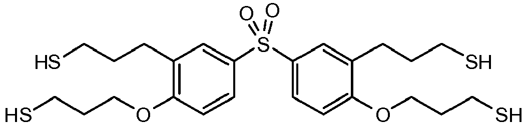

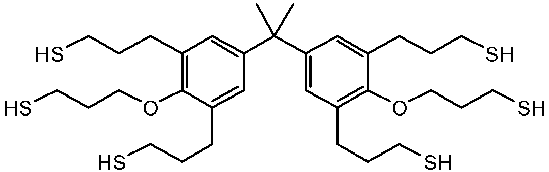

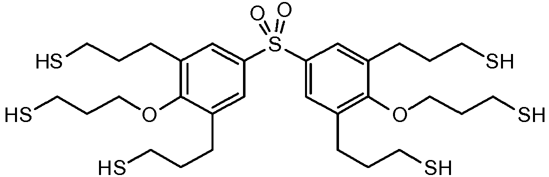

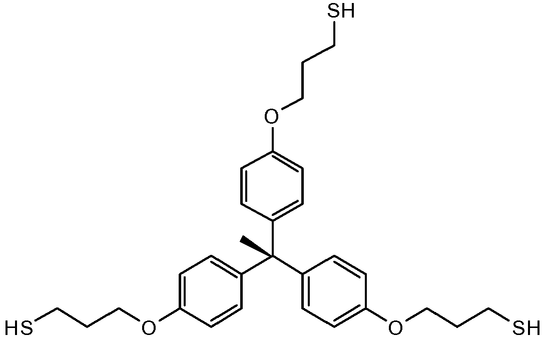

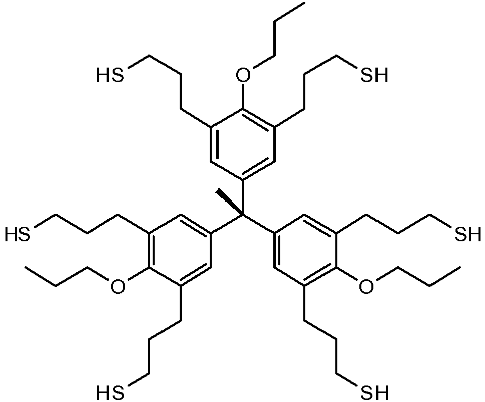

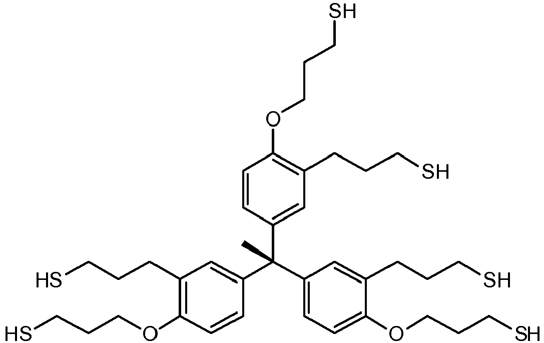

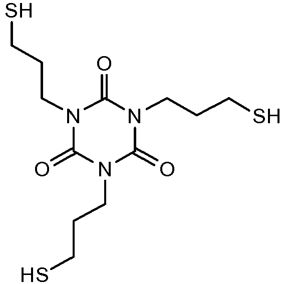

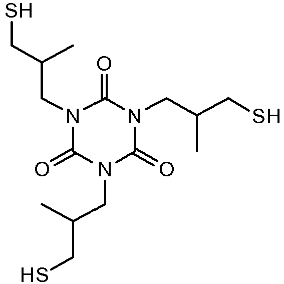

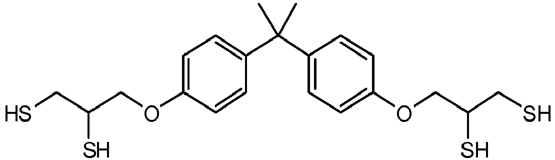

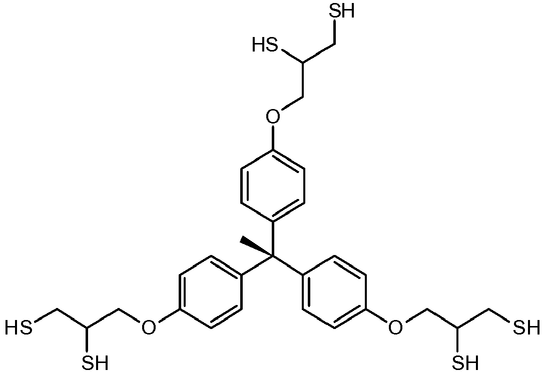

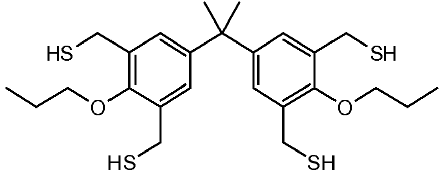

Gemäß einer besonders bevorzugten Ausführungsform der Erfindung werden die Verbindungen der Formel (I) nicht direkt zur Herstellung von Dentalwerkstoffen verwendet, sondern in Form eines Reaktionsprodukts, z.B. mit einem di- oder multifunktionellen Acrylat oder Acrylamid oder mit einem di- oder multifunktionellen Isocyanat, eingesetzt. Hierzu wird das Thiol der Formal (I) in oligomere Verbindungen überführt, die dann als Thiolkomponente zur Herstellung von Dentalwerkstoffen verwendet werden.

Vorzugsweise erfolgt die Oligomerbildung durch eine nukleophile Thiol-En-Reaktion oder Thiol-Isocyanat-Reaktion. Dabei handelt es sich um Polyadditionen, bei denen das Thiol der Formel (I) im stöchiometrischen Überschuss bezogen auf die funktionellen Gruppen mit einer di- oder multifunktionellen En-Verbindung oder einem di- oder multifunktionellen Isocyanat umgesetzt wird. Unter di- oder multifunktionellen En-Verbindungen bzw. Isocyanaten werden Verbindungen mit zwei oder mehr C-C-Mehrfachbindungen bzw. Isocyanatgruppen verstanden. Die oligomeren Thiole weisen drei oder mehr, vorzugsweise 4 bis 10 Mercaptogruppen auf.

Die Oligomermolmasse kann entsprechend der Formel Pₙ = (1+1/r)/(1-2p+1/r) mit dem Reaktionsumsatz erhöht oder verringert werden, wobei Pₙ = der mittlere Polyadditionsgrad, r = das molare Ausgangsverhältnis der Thiol- und En-Gruppen bzw. Isocyanatgruppen und p = der Reaktionsumsatz ist (100% Umsatz: p = 1). Bevorzugt sind Oligomere mit einer zahlenmittleren Molmasse von 700 bis 9.000 g/mol. Im Falle von Reaktionsmischungen mit einer Funktionalität > 2 kann es bei der Polyaddition zur Gelbildung kommen, wobei der Umsatz, bei dem die Gelbildung einsetzt, vom stöchiometrischen Verhältnis der funktionellen Gruppen und der Funktionalität der Reaktionsmischung abhängt. Die Gelbildung tritt beim Einsatz von höherfunktionalisierten Mischungen und annähernd stöchiometrischen Verhältnis der funktionellen Gruppen früher auf. Dementsprechend ist es für die Oligomerbildung vorteilhaft, einen deutlichen Überschuss an SH-Gruppen einzusetzen.

Ein Verfahren zur Herstellung des oligomeren Thiols, bei dem man ein Thiol der Formel (I) im stöchiometrischen Überschuss mit einer di- oder multifunktionellen En-Verbindung, vorzugsweise einem di- oder multifunktionellen Acrylat oder Acrylamid, oder mit einem di- oder multifunktionellen Isocyanat umsetzt, ist ebenfalls Gegenstand der Erfindung. Vorzugsweise wird das Thiol der Formel (I) mit einem di- oder multifunktionellen Acrylat, di- oder multifunktionellen Acrylamid oder di- oder multifunktionellen Isocyanat in einem molaren Verhältnis von SHzu Acryl- bzw. NCO-Gruppen von 1,5 bis 15 : 1, vorzugsweise 1,5 bis 9 : 1 umsetzt.

Als En-Komponente für die Oligomerbildung sind Verbindungen mit elektronenarmen Mehrfachbindungen bevorzugt, wie z.B. di- oder multifunktionelle Acrylate und Acrylamide, weil π-elektronenarme Acrylate und Acrylamide ebenso wie Isocyanate in einer schnellen nucleophilen Polyaddition Oligomere bilden. Elektronenarme Mehrfachbindungen sind daher besonders als En-Komponente für die nucleophile Thiol-En-Reaktion geeignet. Elektronenarme Mehrfachbindungen sind solche, die mit elektronenziehenden Gruppen (-M/-I-Effekt) verbunden sind.

Bevorzugte En-Verbindungen für die Oligomerbildung sind Diacrylate wie Bisphenol-A-diacrylat, Bis-GA (ein Additionsprodukt aus Acrylsäure und Bisphenol-A-diglycidylether), ethoxy- oder propoxyliertes Bisphenol-A-Diacrylat, UDA (ein Additionsprodukt aus 2-Hydroxyethylacrylat und 2,2,4-Trimethylhexamethylendiisocyanat), Di-, Tri- oder Tetraethylenglycoldiacrylat, 1,4-Butandioldiacrylat, 1,10-Decandioldiacrylat oder 1,12-Dodecandioldiacrylat. Bevorzugte Isocyanate sind Hexamethylendiisocyanat und 2,2,4-Trimethylhexamethylendiisocyanat.

Oligomere Mercaptoverbindungen lassen sich beispielsweise durch literaturbekannte nucleophile Thiol-En-Reaktionen von di- oder multifunktionellen Acrylaten oder Acrylamiden mit tri- oder höherfunktionalisierten Mercaptoverbindungen synthetisieren (vgl. J. W. Chan, C. E. Hoyle, A. B. Lowe, M. Bowman, Macromolecules 43 (2010) 6381-6388; G-Z. Li, R. K. Randex, A. H. Soeriyadi, G. Rees, C. Boyer, Z. Tong, Polym. Chem. 1 (2010) 1196-1204; B. D. Mather, K. Viswanathan, K. M. Miller, T. E. Long, Prog. Polym. Sci. 31 (2006) 487-531).

### Allgemeines Beispiel:

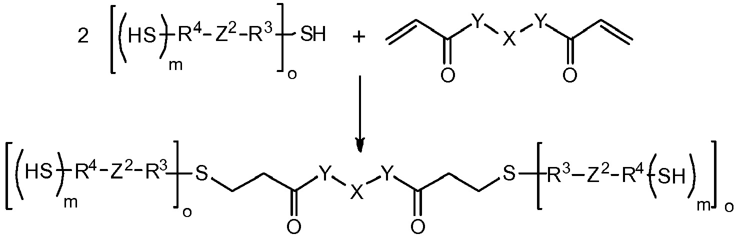

Ein konkretes Beispiel ist:

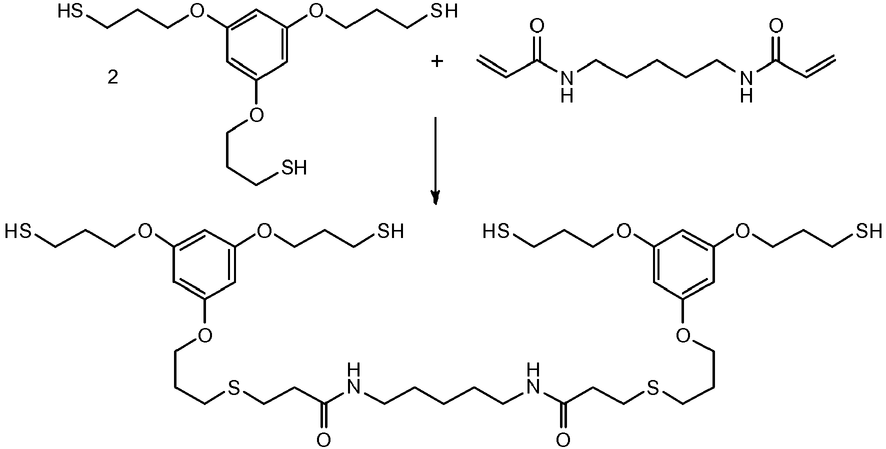

Durch literaturbekannte nucleophile Thiol-Isocyanat-Reaktion von di- oder multifunktionellen Isocyanaten mit tri- oder höherfunktionalisierten Mercaptoverbindungen lassen sich ebenfalls oligomere Mercaptoverbindungen synthetisieren (vgl. H. Li., B. Yu, H. Matsushima, C. E. Hoyle, A. B. Lowe, Macromolecules 42 (2009) 6537-6542).

Ein konkretes Beispiel ist:

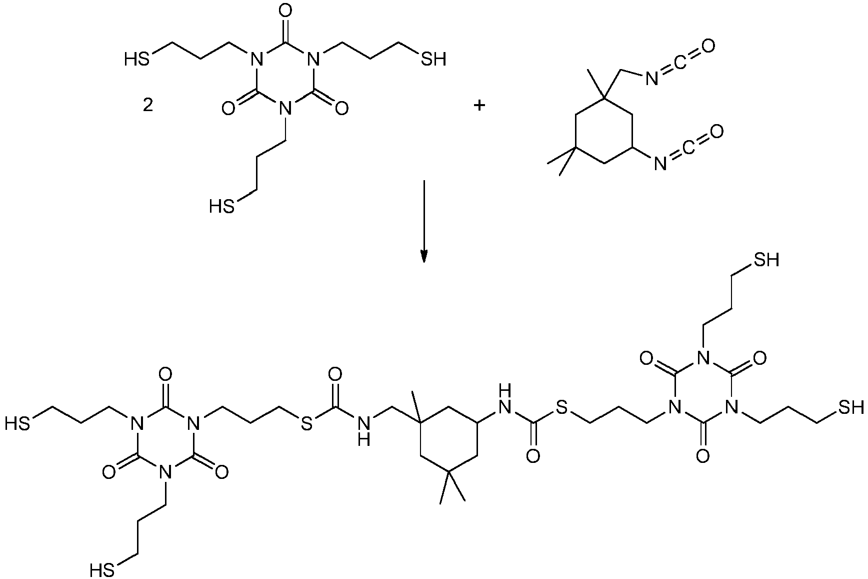

Zur Herstellung von Dentalwerkstoffen werden ein oder mehrere Thiole der Formel (I) oder darauf basierende oligomere Thiole mit mindestens einer Verbindung kombiniert, die drei oder mehr C-C-Mehrfachbindungen enthält (En-Komponente). Hier sind als En-Komponente Verbindungen mit elektronenreichen Mehrfachbindungen bevorzugt, wie z.B. π-elektronenreiche Vinyl-, Allyl- oder Norbornenverbindungen sowie Alkine. Elektronenreiche En-Verbindungen ergeben mit SH-Verbindungen lagerstabile Mischungen und lassen sich durch radikalische Polyaddition schnell aushärten. Elektronenreiche Mehrfachbindungen sind solche, die mit elektronenschiebenden Gruppen (+M/+I-Effekt) verbunden sind.

Als elektronenreiche Vinylverbindungen eigen sich vor allem Vinylether, Vinylester und N-Vinylamide, beispielsweise Trimethylolpropantrivinylether oder Pentaerythrittetravinylether.

Als elektronenreiche Allylverbindungen lassen sich u.a. Allylether von tri- oder höherfunktionalisierten Alkoholen einsetzen, wie beispielsweise Trimethylolpropantriallylether oder Pentaerythrittetraallylether. Geeignet sind auch z.B. Umsetzungsprodukte von tri- oder höherfunktionalisierten Carbonsäuren mit Allylalkohol oder Allylamin oder auch anderen tri- oder höherfunktionalisierten Allylverbindungen wie z.B. Triallylamin oder Triallyl-1,3,5-triazin-2,4,6-trion (TATATO).

Als Norbornen-Verbindungen lassen sich u.a. die Ester von 5-Norbornen-2-carbonsäure mit tri- oder höherfunktionalisierten Alkoholen oder Ester von 5-Norbornen-2-methanol mit tri- oder höherfunktionalisierten Carbonsäuren verwenden. Beispiele sind Trimethylolpropantrioltri-(5-norbonen-2-carbonsäure)ester oder Benzol-1,3,5-tricarbonsäuretri-(5-norbornen-2-methanol)ester.

Als tri- oder höherfunktionalisierte Alkine können u.a. Ester von Propargylalkohol mit tri- oder höherfunktionalisierten Carbonsäuren oder Ether von Propargylalkohol mit tri- oder höherfunktionalisierten Alkoholen eingesetzt werden. Beispiele sind Benzol-1,3,5-tricarbonsäuretripropargylester oder Pentaerythrittetrapropargylether.

Besonders bevorzugte elektronenreiche En-Verbindungen sind tri-oder höherfunktionalisierte Allylverbindungen, vorzugsweise Triallyl-1,3,5-triazin-2,4,6-trion (TATATO).

Darüber hinaus können die erfindungsgemäßen Dentalwerkstoffe vorzugsweise auch mono- oder multifunktionelle Methacrylate oder Mischungen davon als Comonomere enthalten. Unter monofunktionellen Methacrylaten werden Verbindungen mit einer, unter polyfunktionellen Methacrylaten Verbindungen mit zwei oder mehr, vorzugsweise 2 bis 4 radikalisch polymerisierbaren Gruppen verstanden. Die Dentalwerkstoffe enthalten vorzugsweise keine freien Acrylate oder Acrylamide, weil diese aufgrund ihrer großen SH-Reaktivität die Lagerstabilität beeinträchtigen können.

Beispiele für mono- oder multifunktionelle Methacrylate sind Methyl-, Ethyl-, 2-Hydroxyethyl-, Butyl-, Benzyl-, Tetrahydrofurfuryl- oder Isobornylmethacrylat, p-Cumyl-phenoxyethylenglycolmethacrylat (CMP-1E), Bisphenol-A-di-methacrylat, Bis-GMA (ein Additionsprodukt aus Methacrylsäure und Bisphenol-A-diglycidylether), ethoxy- oder propoxyliertes Bisphenol-A-Dimethacrylat, wie z.B. das Bisphenol-A-Dimethacrylat SR-348c (Sartomer) mit 3 Ethoxygruppen oder 2,2-Bis[4-(2-methacryl-oxypropoxy)phenyl]propan, UDMA (ein Additionsprodukt aus 2-Hydroxyethylmethacrylat und 2,2,4-Trimethylhexamethylendiisocyanat), Di-, Tri- oder Tetraethylenglycoldimethacrylat, Trimethylolpropantrimethacrylat, Pentaerythrittetramethacrylat, sowie Glycerindi- und trimethacrylat, 1,4-Butandioldimethacrylat, 1,10-Decandioldimethacrylat (D₃MA) oder 1,12-Dodecandioldimethacrylat.

Die erfindungsgemäßen Dentalwerkstoffe enthalten eine Monoacyltrialkyl- oder Diacyldialkylgermanium-Verbindung als Photoinitiator, vorzugsweise Benzoyltrimethylgermanium, Dibenzoyldiethylgermanium oder Bis(4-methoxybenzoyl)diethylgermanium. Dabei lassen sich auch Mischungen der verschiedenen Photoinitiatoren einsetzen, wie z.B. Bis(4-methoxybenzoyl)diethylgermanium in Kombination mit Campherchinon und 4-Dimethylaminobenzoesäureethylester.

Gemäß einer bevorzugten Ausführungsform enthalten die erfindungsgemäßen Dentalwerkstoffe zusätzlich organischen oder vorzugsweise anorganischen partikulären Füllstoff.

Besonders geeignet sind Füllstoffe auf der Basis von Oxiden mit einer Partikelgröße von 0,1 bis 1,5 µm, wie SiO₂, ZrO₂ und TiO₂ bzw. Mischoxiden aus SiO₂, ZrO₂, ZnO und/oder TiO₂, nanopartikuläre oder mikrofeine Füllstoffe mit einer Partikelgröße von 0,01 bis 500 nm, wie pyrogene Kieselsäure oder Fällungskieselsäure sowie Glaspulver mit einer Partikelgröße von 0,01 bis 15 µm, vorzugweise von 0,2 bis 1,5 µm, wie Quarz-, Glaskeramik-oder röntgenopake Glaspulver von z.B. Barium- oder Strontiumaluminiumsilikatgläsern, und röntgenopake Füllstoffe mit einer Partikelgröße von 0,2 bis 1,5 µm, wie Ytterbiumtrifluorid, Tantal(V)-oxid, Bariumsulfat oder Mischoxide von SiO₂ mit Ytterbium(III)-oxid oder Tantal(V)-oxid. Auch faserförmige Füllstoffe, Nanofasern oder Whiskers sind nicht ausgeschlossen. Wenn nicht anders angegeben handelt es sich bei allen Partikelgrößen um gewichtsmittlere Partikelgrößen.

Die Füllstoffe werden nach der Partikelgröße unterteilt in Makrofüller und Mikrofüller. Makrofüller werden durch Mahlen von Quarz, röntgenopaken Gläsern, Borosilikaten oder von Keramik gewonnen, sind rein anorganischer Natur und bestehen meist aus splitterförmigen Teilen. Bevorzugt sind Makrofüller mit einer mittleren Partikelgröße von 0,2 bis 10 µm. Als Mikrofüller werden vorzugsweise pyrogenes SiO₂ oder Fällungskieselsäure eingesetzt, oder auch Mischoxide, z.B. SiO₂-ZrO₂, die durch hydrolytische Cokondensation von Metallalkoxiden zugänglich sind. Die Mikrofüller haben vorzugsweise eine mittlere Partikelgröße von ca. 5 bis 100 nm.

Die erfindungsgemäßen Dentalwerkstoffe können auch sogenannte Mikrofüller-Komplexe enthalten. Beispiele hierfür sind splitterförmige mikrogefüllte Polymerisate, die z.B. durch Einarbeiten von anorganischen Füllstoffen wie z.B. pyrogenem SiO₂ in eine Harzmatrix, anschließende thermische Polymerisation der Mischung und Mahlen des so erhaltenen Polymerisates zugänglich sind.

Faserförmige Füllstoffe werden insbesondere zur Herstellung von Gerüstmaterialien eingesetzt.

Zur Verbesserung des Verbundes zwischen den Füllstoffpartikeln und der vernetzten Polyadditionsmatrix können SiO₂-basierende Füllstoffe mit Thiol-, Vinyl-, Allyl-, Norborn-2-enyl-, Methacryl- oder Alkin-funktionalisierten Silanen oberflächenmodifiziert werden. Beispiele für solche Silane sind 3-Thiopropyltrimethoxysilan, 3-Allyltriethoxysilan, 3-Methacryloxypropyltrimethoxysilan oder N-[3-(Triethoxysilyl)-propyl]-carbaminsäurepropargylester. Zur Oberflächenmodifizierung von nichtsilikatischen Füllstoffen wie z.B. von ZrO₂ oder TiO₂ können auch funktionalisierte saure Phosphate, wie z.B. Allyl-oder Propargyldihydrogenphosphat eingesetzt werden.

Der Füllgrad richtet sich nach dem gewünschten Anwendungszweck. Füllungskomposite haben vorzugsweise einen Füllstoffgehalt von 75-90 Gew.-% und Kompositzemente von 50-75 Gew.-%.

Gegebenenfalls können die erfindungsgemäß eingesetzten Zusammensetzungen weitere Additive enthalten, vor allem Stabilisatoren, Farbmittel, mikrobiocide Wirkstoffe, fluoridionenabgebende Additive, optische Aufheller, Weichmacher und/oder UV-Absorber, insbesondere Inhibitoren, UV-Stabilisatoren und Pigmente.

Dabei sind erfindungsgemäß Dentalmaterialien bevorzugt, welche die folgenden Komponenten enthalten:
a) 5 bis 40 Gew.-%, bevorzugt 5 bis 30 Gew.-% und besonders bevorzugt 5 bis 20 Gew.-% Thiol der allgemeinen Formel I oder ein Oligomer davon,
b) 5 bis 40 Gew.-%, bevorzugt 5 bis 30 Gew.-% und besonders bevorzugt 5 bis 20 Gew.-% En-Komponente,
c) 0 bis 40 Gew.-%, bevorzugt 2 bis 30 Gew.-% und besonders bevorzugt 4 bis 20 Gew.-% Methacrylat(e),
d) 0,01 bis 10 Gew.-%, besonders bevorzugt 0,1 bis 3,0 Gew.-% an Initiator,
e) 10 bis 85 Gew.-% Füllstoff, und
f) 0 bis 10 Gew.-%, bevorzugt 0 bis 5 Gew.-% Additive.

Besonders bevorzugt sind solche Dentalwerkstoffe, welche aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind. Besonders bevorzugt sind Werkstoffe, die neben dem Thiol der Formel (I) oder einem Oligomer davon keine flüchtigen Mercaptane enthalten, d.h. Mercaptane, die einen typischen Mercaptangeruch aufweisen. Ganz besonders bevorzugt sind Zusammensetzungen, die keine weiteren Mercaptane und vorzugsweise auch keine anderen Schwefelverbindungen enthalten.

Die erfindungsgemäßen Dentalwerkstoffe eignen sich besonders als dentale Zemente, Füllungskomposite und Verblendmaterialien sowie als Materialien zur Herstellung von Inlays, Onlays, Kronen und Brücken. Sie zeichnen sich durch einen hohen Umsatz der polyreaktionsfähigen Gruppen, ähnliche mechanische Eigenschaften wie auf Dimethacrylaten basierende Materialien, eine verringerte Polymerisationsspannung, geringen Eigengeruch und geruchsstabile Eigenschaften auch nach langer Lagerung aus. Außerdem weisen sie einen geringen Polymerisationsschrumpf auf.

Die Dentalwerkstoffe eignen sich primär zur intraoralen Anwendung durch den Zahnarzt zur Restauration geschädigter Zähne (klinische Materialien). Sie können aber auch extraoral eingesetzt werden, beispielsweise bei der Herstellung oder Reparatur von Dentalrestaurationen (technische Materialien).

Die Erfindung wird im Folgenden anhand von Ausführungsbeispielen näher erläutert.

### Ausführungsbeispiele

### Beispiel 1:

### Synthese eines tetrathiolfunktionalisierten Bisphenol A Derivats durch radikalische Addition von Thioessigsäure an einen Allylprecursor

### a) Synthese von 2,2-Bis[3-(3-acetylmercaptopropyl)-4-(3-acetylmercapto-propoxy)-phenyl]propan

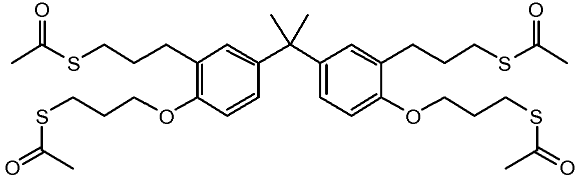

In einem 500 mL Dreihalskolben wurden 29,14 g (75 mmol) 2,2-Bis[3-allyl-4-allyloxyphenyl]propan, welches nach literaturbekannten Vorschriften (vgl. Patent WO 98/58294 A1; M. Abraham, I. Hamerton, J. Rose, J. Grate, J. Chem. Soc. Perkin Trans. 2 (1991) 1417-1423) aus Bisphenol A und Allylbromid mittels Williamson Ethersynthese und Claisen-Umlagerung zugänglich ist, 34,25 g (450 mmol) Thioessigsäure und 2,46 g (15 mmol) 2,2'-Azobis(2-methylpropionnitril) in 200 mL Tetrahydrofuran vorgelegt. Die Reaktionslösung wurde 30 min mit Stickstoff gespült und anschließend unter Stickstoffatmosphäre für 16 Stunden bei 65 °C gerührt. Nach dem Abkühlen der Reaktionslösung im Eisbad wurden 100 mL einer ein molaren Natriumcarbonat-Lösung hinzugetropft. Nach dreifacher Extraktion mit Dichlormethan wurden die vereinten organischen Phasen zweimal mit einer ein molaren Natriumhydroxid-Lösung sowie gesättigter Natriumchlorid-Lösung gewaschen, über Magnesiumsulfat getrocknet und vom Lösungsmittel am Rotationsverdampfer unter reduziertem Druck befreit. Das Rohprodukt wurde säulenchromatographisch über Kieselgel gereinigt, um 35,8 g (51,7 mmol, 69 % der Theorie) 2,2-Bis[3 -(3-acetylmercaptopropyl)-4-(3-acetylmercapto-propoxy)-phenyl]-propan als gelbes hochviskoses Öl zu erhalten.
¹H NMR (300 MHz, CDCl₃, δ [*ppm*]): 7,02 - 6,92 (m, 4H, Ar***H***), 6,69 (d, ³J_{HH}= 8,5 Hz, 2H, Ar***H***), 3,98 (t, ³J_{HH}=5,9 Hz, 4H, OC***H*₂**), 3,07 (t, ³J_{HH}=7,1 Hz, 4H, C***H₂***S), 2,85 (t, ³J_{HH}=7,2 Hz, 4H, C***H₂***S), 2,63 (m, 4H, ArC***H₂***), 2,34 and 2,32 (s, 12H, C***H₃***), 2,14 - 2,01 (m, 4H, C***H₂***), 1,90 -1,75 (m, 4H, C***H₂***), 1,61 (s, 6H, C(C***H₃***)₂) ;
¹³C NMR (75 MHz, CDCl₃, δ *[ppm]):* 196,01 and 195,81 (S(***C***=O)CH₃), 154,50 (Ar***C***), 143,08 (Ar***C***), 128,98 (Ar***C***), 128,90 (Ar***C***), 125,39 (Ar***C***), 110,48 (Ar***C***), 66,12 (ArO***C***H₂), 41,75 (***C***(CH₃)₂), 31,26 (C(***C***H₃)₂), 30,80 (S(C=O)***C***H₃), 29,98 (***C***H₂), 29,83 (***C***H₂), 29,64 (***C***H₂), 29,05 (***C***H₂), 26,24 (***C***H₂);
FT-IR: v = 2962 (w), 2926 (w), 2867 (w), 1685 (s, v_{C=O}), 1607 (w), 1500 (s), 1470 (m), 1415 (m), 1410 (m), 1383 (w), 1353 (m), 1294 (w), 1243 (s), 1131 (s), 1105 (s), 1038 (m), 953 (s) cm⁻¹;
MALDI-TOF-MS: *m*/*z_{gef}:* 692.2 (M⁺), 715.2 (M+Na⁺), 731.2 (M+K⁺), *m*/*z_{ber}:* 715.22 (M+Na⁺).

### b) Synthese von 2,2-Bis[3-(3-mercaptopropyl)-4-(3-mercaptopropoxy)phenyl]-propan

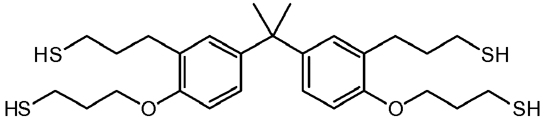

In einem 500 mL Dreihalskolben wurden 35,69 g (51,50 mmol) 2,2-Bis[3-(3-acetylmercaptopropyl)-4-(3-acetylmercapto-propoxy)-phenyl]propan, 22,17 g konz. Salzsäure (37 Gew%) in einer Mischung aus 200 mL Methanol und 50 mL Tetrahydrofuran vorgelegt. Die Reaktionslösung wurde 30 min mit Stickstoff durchspült und anschließend für 20 Stunden bei 60 °C unter einer Stickstoffatmosphäre gerührt. Nach dem Abkühlen auf Raumtemperatur wurden 150 mL destilliertes Wasser hinzugegeben. Nach dreifacher Extraktion mit Dichlormethan wurden die vereinten organischen Phasen zweimal mit einer ein molaren Natriumhydroxid-Lösung sowie gesättigter Natriumchlorid-Lösung gewaschen, über Magnesiumsulfat getrocknet und vom Lösungsmittel am Rotationsverdampfer unter reduziertem Druck befreit. Das Rohprodukt wurde säulenchromatographisch über Kiesegel gereinigt, um nach Trocknung im Feinvakuum 20,8 g (39,6 mmol, 76 % der Theorie) 2,2-Bis[3 -(3-mercaptopropyl)-4-(3-mercaptopropoxy)-phenyl]propan als hochviskoses Öl zu erhalten.
¹H NMR (300 MHz, CDCl₃, δ [*ppm*]): 7,02 (dd, ³J_{HH}=8,6 Hz / ⁴J_{HH}=2,5 Hz, 2H, Ar***H***), 6,94 (d, ⁴J_{HH}=2,5 Hz, 2H, Ar***H***), 6,73 (d, ³J_{HH}=8,5 Hz, 2H, Ar***H***), 4,04 (t, ³J_{HH}=5,8 Hz, 4H, OC***H₂***), 2,79-2,70 (m, 4H, C***H₂***SH), 2,69 - 2,61 (m, 4H, ArC***H₂***), 2,53 - 2,42 (m, 4H, C***H₂***SH), 2,14 - 2,03 (m, 4H, C***H₂***), 1,90 - 1,77 (m, 4H, C***H₂***), 1,61 (s, 6H, C(C***H₃***)₂), 1,40 (t, ³J_{HH}=8.1 Hz, 2H, S***H***), 1,34 (t, ³J_{HH}=7,8 Hz, 2H, S***H***) ;
¹³C NMR (75 MHz, CDCl₃, δ *[ppm]):* 154,54 (Ar***C***), 143,05 (Ar***C***), 129,08 (Ar***C***), 129,04 (Ar***C***), 125,22 (Ar***C***), 110,52 (Ar***C***), 65,55 (O***C***H₂), 41,74 (***C***(CH₃)₂), 34,30 (***C***H₂), 33,65 (***C***H₂), 31,26 (C(***C***H₃)₂), 29,42 (***C***H₂), 24,45 (***C***H₂), 21,62 (***C***H₂);
FT-IR: v = 3050 (w), 3025 (w), 2961 (m), 2925 (m), 2867 (w), 2560 (w, *v*_{SH}), 1606 (w), 1499 (s), 1468 (m), 1439 (m), 1383 (w), 1360 (w), 1294 (m), 1242 (s), 1181 (m), 1154 (m), 1117 (m), 1034 (m), 810 (s) cm⁻¹; MS (EI) *m*/*z* (%) : 525 (12) [M⁺], 524 (36) [M⁺], 511 (21), 510 (30), 509 (100), 450 (18), 437 (13), 436 (20), 435 (78), 362 (17), 361 (76), 327 (12), 287 (25), 213 (19), 209 (28), 207 (10), 193 (18), 179 (12), 175 (34), 159 (26), 147 (35), 135 (21), 133 (11), 119 (16), 107 (12), 75 (12), 47 (14), 41 (19);
Elementaranalyse
berechnet für C₂₇H₄₀O₂S₄: C 61,79, H 7,68, S 24,44;
gefunden: C 61,99, H 7,62, S 24,65.

### Beispiel 2:

### Synthese von eines tetrathiolfunktionalisierten Bisphenol A Derivats durch Nutzung der radikalischen Addition von Thioessigsäure an Propargyl-Gruppen

### a) Synthese von 2,2-Bis[4-(2,3-diacetylmercaptopropoxy)-phenyl]propan

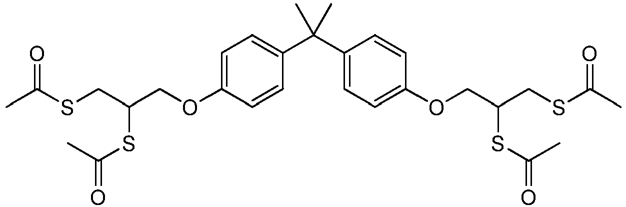

In einem 250 mL Dreihalskolben wurden 10,65 g (35 mmol) 2,2-Bis[4-(prop-2-yn-1-yloxy)phenyl]propan, welches durch eine Williamson-Ethersynthese aus Bisphenol A und Propargylbromid zugänglich ist, 26,64 g (350 mmol) Thioessigsäure, 1,38 g (8,4 mmol) 2,2'-Azobis(2-methylpropionnitril) und 150 mL Toluol vorgelegt. Die Reaktionslösung wurde für 30 min mit Stickstoff durchspült und dann für 24 Stunden bei 65 °C unter einer permanenten Stickstoffatmosphäre gerührt. Nach Entfernung der flüchtigen Bestandteile unter reduziertem Druck wurde das Rohprodukt säulenchromatographisch über Kieselgel gereinigt. Auf diesem Weg wurden 16,0 g (26,3 mmol, 75% der Theorie) 2,2-Bis[4-(2,3-diacetylmercapto-propoxy)phenyl]propan als hochviskoses gelbes Öl erhalten.
¹H NMR (300 MHz, CDCl₃, δ [*ppm*]): 7,10 (d, ³J_{HH}=8,9 Hz, 4H, Ar-***H***), 6,78 (d, ³J_{HH}=8,9 Hz, 4H, Ar-***H***), 4,20 - 4,10 (m, 2H, -C***H***-), 4,05 - 3,94 (m, 4H, -OC***H₂***-), 3,45 - 3,19 (m, 4H, -C***H₂***S-), 2,33 (s, 3H, -*C**H**₃*), 2,32 (s, 3H, -C***H**₃*), 1,60 (s, 6H, -C(C***H***₃)₂);
¹³C NMR (75 MHz, CDCl₃, δ [*ppm*]): 194,70 (-S(***C***=O)CH₃), 194,61 (-S(***C***=O)CH₃), 156,25 (Ar-***C***4), 143,85 (Ar-***C***1), 127,92 (Ar-***C***2,***C***6), 114,16 (Ar-***C***3, ***C***5), 68,73 (Ar-O***C***H₂-), 43,52 (-***C***H-), 41,88 (-***C***(CH₃)₂), 31,16 (-***C***H₂S-), 30,79 (-C(***C***H₃)₂), 30,68 (-***C***H₃), 30,58 (-***C***H₃) ;
FT-IR: v = 3035 (m), 2966 (m), 2930 (m), 2861 (m), 1688 (s, *v*_{C=O}), 1607 (m, *v*_{C=C}), 1582 (w, *v*_{C=C}), 1508 (s, *v*_{C=C}), 1463(m), 1410 (m), 1383 (m), 1353 (m), 1297 (m), 1237 (s), 1181 (s), 1127 (s), 1105 (s) cm⁻¹ ;
MALDI-TOF-MS: m/z_{gef}: 631,1 [M+Na⁺] ; m/z_{ber}: 631,13 [M+Na⁺].

### b) Synthese von 2,2-Bis[4-(2,3-dimercaptopropoxy)phenyl]propan

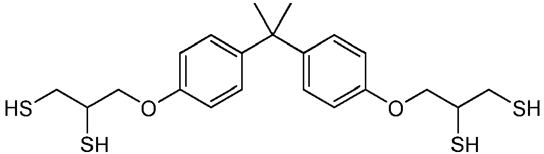

14,92 g (24,50 mmol) 2,2-Bis[4-(2,3-diactylmercaptopropoxy)-phenyl]propan wurden in 40 mL THF in einem 250 mL Zweihalskolben gelöst und permanent mit Stickstoff durchspült. Im Eisbad bei 0 °C erfolgte die tropfenweise Zugabe von 60 mL einer Kalium-Methoxid-Lösung (25 Gew%). Nach beendeter Zugabe wurde die Lösung noch weitere 30 min im Eisbad gerührt und dann in 100 mL einer 1N eiskalten HCl-Lösung eingetragen. Die Suspension wurde in einen Scheidetrichter überführt und dreimal mit Dichlormethan ausgeschüttelt. Die vereinten organischen Phasen wurden mit 100 mL gesättigter Natriumchlorid-Lösung gewaschen, über MgSO₄ getrocknet und vom Lösungsmittel befreit. Das Rohprodukt wurde säulenchromatographisch über Kieselgel gereinigt, um 6,5 g (14,7 mmol, 60% der Theorie) 2,2-Bis[4-(2,3-dimercaptoprop-oxy)phenyl]propan als farblosen Feststoff zu erhalten. Schmelzpunkt: 85 °C;
¹H NMR (300 MHz, CDCl₃, δ *[ppm]):* 7,13 (d, ³J_{HH}=8,9 Hz, 4H, Ar-***H***), 6,80 (d, ³J_{HH}=8,9 Hz, 4H, Ar-***H***), 4,17 (dd, ³J_{HH}=4,7 Hz/ ²J_{HH}=9,6 Hz, 2H, Ar-OC***H***H-), 4,02 (dd, ³J_{HH}=7,3 Hz/ ²J_{HH}=9,6 Hz, 2H, Ar-OC***H***H-), 3,32 - 3,20 (m, 2H, -C***H***-), 3,00 - 2,92 (m, 4H,-C***H₂***SH), 1,95 (d, ³J_{HH}=9,2 Hz, 2H, -S***H***), 1,65 - 1,56 (m, 8H,-C(C***H***₃)₂ und -CH₂S***H***) ;
¹³C NMR (75 MHz, CDCl₃, δ *[ppm]):* 156,23 (Ar-***C***4), 143,91 (Ar-***C***1), 128,01 (Ar-***C***2,***C***6), 114,21 (Ar-***C***3,***C***5), 70,19 (Ar-O***C***H₂-), 41,93 (-***C***(CH₃)₂), 41,70 (-***C***H-), 31,21 (-C(***C***H₃)₂), 29,98 (-***C***H₂SH) ;
FT-IR: v = 3060 (w), 3036 (w), 2962 (m), 2932 (m), 2866 (m), 2557 (m, *v*_{SH}), 1607 (m), 1581 (w), 1508 (s), 1455 (s), 1414 (m), 1378 (m), 1362 (m), 1300 (s), 1232 (s), 1180 (s) cm⁻¹;
MS (EI) m/z (%):440 (3) [M⁺], 228 (32), 214 (15), 213 (100), 135 (12), 73 (13).

### Beispiel 3:

### Synthese eines Thiol-Harzes durch Addition eines trithiolfunktionellen Precursors an 1,4-Butandioldiacryat oder Tricyclo[5.2.1.0(2.6)]decandimethylol-diacrylat (TCD-DA)

### a) Synthese von 1,3,5-Tris(3-acetylmercaptopropyl)-1,3,5-triazin-2,4,6-trion

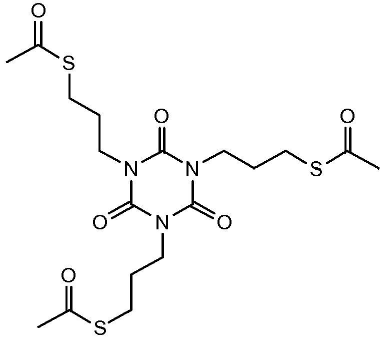

In einem 500 mL Dreihalskolben wurden 37,39 g (150 mmol) 1,3,5-Triallyl-1,3,5-triazin-2,4,6-trion, 41,10 g (540 mmol) Thioessigsäure und 3,69 g (22,5 mmol) 2,2'-Azobis(2-methylpropionnitril) in 250 mL Tetrahydrofuran analog der US 4,266,055 gelöst. Die Reaktionslösung wurde 30 min mit Stickstoff durchspült und anschließend unter Stickstoffatmosphäre für 16 Stunden bei 65 °C erhitzt. Nach dem Abkühlen der Reaktionslösung im Eisbad auf 0 °C wurden 100 mL einer ein molaren Natriumcarbonat-Lösung hinzugetropft. Nach dreifacher Extraktion mit Dichlormethan wurden die vereinten organischen Phasen mit 80 mL einer ein molaren Natriumhydroxid-Lösung sowie gesättigter Natriumchlorid-Lösung gewaschen, über Magnesiumsulfat getrocknet und vom Lösungsmittel am Rotationsverdampfer unter reduziertem Druck befreit. Das Rohprodukt wurde dreimal aus 200 mL Methanol umkristallisiert um 1,3,5-Tris(3-acetylmercaptopropyl)-1,3,5-triazin-2,4,6-trion (48,0 g, 100,5 mmol, 67% der Theorie) als einen farb- und geruchslosen Feststoffs zu erhalten. Schmelzpunkt: 66 - 67 °C;
¹H NMR (300 MHz, CDCl₃, δ [*ppm*]): 3,89 (t, ³J_{HH}=7,1 Hz, 6H,-NC***H₂***-), 2,83 (t, ³J_{HH}=7,1 Hz, 6H, -C***H₂***S-), 2,26 (s, 9H, -C***H**₃*), 1,87 (quin., ³J_{HH}=7,1 Hz, 6H, -C***H₂***-) ;
¹³C NMR (75 MHz, CDCl₃, δ [*ppm*]): 195,45 (-S(***C***=O)CH₃), 149,03 (-***C***=O)*,* 42,07 (-N*C*H₂-), 30,68 (-***C***H₃), 28,03 (-***C***H₂-) , 26,27 (-***C***H₂S-) ;
FT-IR: v= 3024 (w), 2977 (m), 2945 (w), 2923 (w), 1692 (s, *v*_{C=O}), 1676 (s, *v*_{C=O}), 1508 (w), 1457 (s), 1425 (s), 1373 (m), 1352 (m), 1338 (m), 1327 (m), 1307 (m), 1296 (w), 1283 (w), 1242 (w), 1135 (s), 1107 (s), 1045 (w), 955 (m), 763 (s, *v*_{C-S}) cm⁻¹;
MS (EI) m/z (%): 519 (4) [M⁺], 477 (16), 476 (32), 444 (12), 434 (14), 402 (26), 400 (12), 390 (11), 360 (24), 358 (22), 348 (42), 326 (12), 314 (12), 306 (50), 272 (40), 184 (16), 130 (23), 96 (10), 87 (19), 56 (15), 55 (17), 43 (100), 41 (10).

### b) Synthese von 1,3,5-Tris(3-mercaptopropyl)-1,3,5-triazin-2,4,6-trion

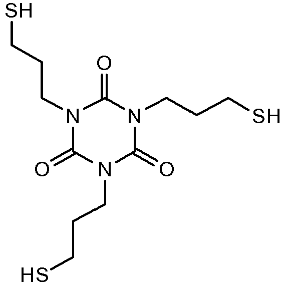

In einem 500 mL Dreihalskolben wurden 44,89 g (94 mmol) 1,3,5-Tris(3-acetyl-mercaptopropyl)-1,3,5-triazin-2,4,6-trion in einer Mischung aus 190 mL Methanol und 60 mL 1,4-Dioxan gelöst. Diese Lösung wurde 30 min mit Stickstoff durchspült und dann erfolgte die Zugabe von 29,43 g konzentrierter Salzsäure-Lösung (37 Gew%). Die Reaktionslösung wurde für 20 Stunden bei 60 °C unter einer Stickstoffatmosphäre gerührt. Nach dem Abkühlen auf Raumtemperatur wurden 200 mL destilliertes Wasser hinzugegeben und die wässrige Phase dreimal mit Dichlormethan extrahiert. Die kombinierten organischen Phasen wurden mit zweimal 100 mL gesättigter Natriumhydrogencarbonat- sowie Natriumchlorid-Lösung gewaschen, über Magnesiumsulfat getrocknet und nach Filtration am Rotationsverdampfer unter reduziertem Druck vom Lösungsmittel befreit. 32,2 g (91,6 mmol, 97% der Theorie) 1,3,5-Tris(3-mercaptopropyl)-1,3,5-triazin-2,4,6-trion wurden als geruchsarmes, farblose Öl erhalten (Nullscherviskosität bei 25 °C: ca. 5 Pa*s). Eine weitere Geruchsreduktion war durch Filtration über Kieselgel möglich.
¹H NMR (300 MHz, CDCl₃, δ *[ppm]):* 4, 01 (t, ³J_{HH}=7,0 Hz, 6H,-NC***H₂***-), 2.56 (dt, ³J_{HH}=6,9 Hz / ³J_{HH}=8,0 Hz, 6H, -C***H₂***S-), 1,97 (quin., ³J_{HH}=7,0 Hz, 6H, -C***H*₂**-), 1,54 (t, ³J_{HH}=8,0 Hz, 6H,-CH₂S***H***) ;
¹³C NMR (75 MHz, CDCl₃, δ *[ppm]):* 149,10 (-***C***=O), 41,91 (-N***C***H₂-), 31,94 (-***C***H₂-), 22,03 (-***C***H₂SH) ;
FT-IR: v= 2963 (w), 2933 (w), 2857 (w), 2568 (w, *v*_{SH}), 1671 (s, *v*_{C=O}), 1502 (w), 1454 (s), 1422 (s), 1374 (m), 1334 (m), 1318 (m), 1288 (m), 1258 (m), 762 (s, *v*_{V-S}) cm⁻¹;
MS (EI) *m*/*z* (%) : 351 (22) [M⁺], 319 (19), 318 (85), 317 (34), 286 (17), 284 (100), 244 (20), 224 (25), 210 (49), 170 (27), 127 (21), 84 (29), 70 (41), 56 (77), 47 (22), 41 (35);
Elementaranalyse
berechnet für C₁₂H₂₁N₃O₃S₃: C 41,00, H 6,02, N 11,95, S 27,37; gefunden: C 41,02, H 5,92, N 11,84, S 27,48.

### c) Thiol-Michael-Addition von 1,4-Butandioldiacrylat und 1,3,5-Tris(3-mercapto-propyl)-1,3,5-triazin-2,4,6-trion

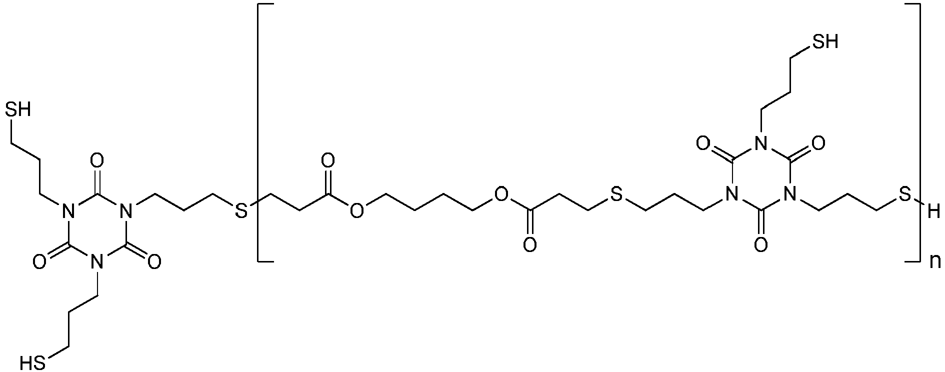

In einer 10 mL Mikrowellendruckviole mit Septum wurden 1,05 g (3 mmol) 1,3,5-Tris(3-mercaptopropyl)-1,3,5-triazin-2,4,6-trion und 148,5 mg (0,75 mmol) 1,4-Butandioldiacrylat unter einer Argonatmosphäre homogenisiert. Es wurden 50 mg Triethylamin als Katalysator hinzugegeben, und das Reaktionsgemisch wurde 24 Stunden bei 50 °C gerührt. Das Triethylamin wurde durch mehrfaches Lösen in Dichlormethan herausgeschleppt. Das Additionsprodukt wurde als farbloses Öl ohne wahrnehmbaren Geruch erhalten. Das Geruchsbild veränderte sich auch nach Lagerung im Kühlschrank über einen Zeitraum von 6 Monaten nicht. Gelpermeationschromatographische Untersuchungen in Tetrahydrofuran als Laufmittel zeigten neben dem noch vorhandenem Monomer 1,3,5-Tris(3-mercaptopropyl)-1,3,5-triazin-2,4,6-trion (n=0), das Dimer (n=1) und Trimer (n=2) als Hauptprodukte neben einem geringem Anteil höherer Homologe an.

### d) Thiol-Michael-Addition von Tricclo[5.2.1.0(2.6)]decandi-methyloldiacrylat (TCD-DA) und 1,3,5-Tris (3-mercaptopropyl)-1,3,5-triazin-2,4,6-trion

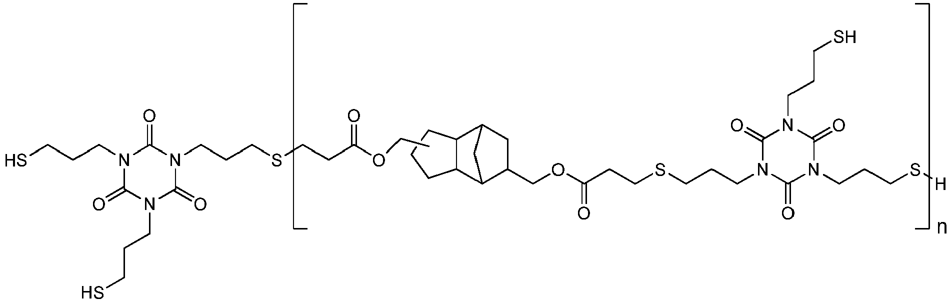

In einem 100 mL Schlenkkolben wurden 12,02 g (34,20 mmol) 1,3,5-Tris(3-mercaptopropyl)-1,3,5-triazin-2,4,6-trion, 2,60 g (8,55 mmol) Tricyclo[5.2.1.0(2.6)]-decandimethylol-diacrylat (TCD-DA) in 25 mL Tetrahydrofuran gelöst. Die Lösung wurde für 30 min mit Stickstoff durchspült, dann erfolgte die Zugabe von 2 mL (1,46 g) Triethylamin. Die Reaktionslösung wurde für 17 Stunden bei 40 °C gerührt und dann in 100 mL einer ein molaren Salzsäure-Lösung eingetragen. Die wässrige Phase wurde dreimal mit Dichlormethan extrahiert. Die vereinten organischen Phasen wurden anschließend mit jeweils zweimal 100 mL einer ein molaren Salzsäure-Lösung und einer gesättigten Natriumchlorid-Lösung gewaschen, über Magnesiumsulfat getrocknet, filtriert und am Rotationsverdampfer unter reduziertem Druck vom Lösungsmittel befreit. Das Additionsprodukt wurde in quantitativen Ausbeuten als nahezu geruchsloses, farbloses Öl erhalten, welches nach Lagerung im Kühlschrank über einen Zeitraum von 4 Monaten keine Geruchsveränderung zeigte. Gelpermeationschromatographische Untersuchungen in Tetrahydrofuran als Laufmittel zeigten neben dem noch vorhandenem Monomer 1,3,5-Tris(3-mercaptopropyl)-1,3,5-triazin-2,4,6-trion (n=0), das Dimer (n=1) und Trimer (n=2) als Hauptprodukte neben einem geringem Anteil höherer Homologe an. (Nullscherviskosität bei 25 °C: ca. 113 Pa*s).

### Beispiel 4:

### Füllungskomposite auf der Basis von Thiol-En-Vernetzern

Entsprechend der nachfolgend aufgeführten Tabelle 1 wurden Komposite Material A und Referenz A und B (Angaben in Masse-%) hergestellt. Dazu wurden die Reaktivkomponenten (Thiol- und En-Komponenten) zusammen mit dem Stabilisator und den Initiator im Zentrifugalmischer Speedmixer (Fa. Hauschild) bei 1000 RPM für 60 s homogenisiert. Zur homogenen Flüssigkeit wurde die Füllstoffmenge in mehreren Portionen mit abnehmender Portionsgröße zugegeben. Nach jeder Zugabe erfolgte eine Homogenisierung bei 1000 RPM für jeweils 60 s. Die Mischung sollte handwarm werden aber nicht heiß. Die finale Kompositpaste wurde nach 6-8 Füllstoffzugaben erreicht. Ein abschließendes Mischen bei 600 RPM für ca. 5 min sorgte für eine blasenfreie homogene Masse.

**Tabelle 1: Zusammensetzung der Komposite**

| **Inhaltsstoff** | **Material A** | **Referenz A*)** | **Referenz B*)** |
|---|---|---|---|
| TATATO¹⁾ | 10,4 | 12,9 | - |
| Produkt aus Bsp. 3d²⁾ | 21,5 | - | - |
| PETMP³⁾ | - | 19,0 | - |
| Bis-GMA | - | - | 22,4 |
| TEGDMA | - | - | 9,6 |
| Glasfüller G018-053 UF1,5 sil⁴⁾ | 67,9 | 67,7 | 67,8 |
| Stabilisator⁵⁾ | 0,1 | 0,2 | 0,1 |
| Photoinitiator⁶⁾ | 0,1 | 0,3 | 0,1 |
| Summe | 100,0 | 100,0 | 100,0 |

| | | | |
|---|---|---|---|
| *) Vergleichsbeispiel ¹⁾ Triallyl-1,3,5-triazin-2,4,6-trion ²⁾ Michael-Addition von Tricyclo[5.2.1.0(2.6)]decandimethyl-ol-diacrylat (TCD-DA) und 1,3,5-Tris(3-mercaptopropyl)-1,3,5-triazin-2,4,6-trion ³⁾ Pentaerythritoltetrakis(3-mercaptopropionat) ⁴⁾ Silanisierter Ba-Al-Borosilikatglasfüller mit einer mittl. Partikelgröße von 1,5 µm ⁵⁾ BHT ⁶⁾ Radikalbildender blaulichtsensibler Photoinitiator: TPO | | | |

Ausgehend von den Kompositpasten erfolgte die Prüfkörperherstellung beginnt mit der sorgfältigen Befüllung der jeweiligen Prüfkörperformen in mehreren Portionen, wobei Luftblasen durch Stopfen vermieden wurden. Die Prüfkörperformen sind in den jeweiligen Normprüfungen und der Fachliteratur beschrieben. Zur Photopolymerisation wurden die Proben mit Blaulicht im Wellenlängenbereich um 460 nm belichtet. In den Beispielen wurden Dentallichtgeräte mit einer Lichtintensität > 850 mW/cm² (Modell Translux Energy, Fa. Heraeus Kulzer GmbH) benutzt, um die Proben im Belichtungsbereich mit jeweils 20 s zu bestrahlen. Die Belichtung erfolgte beidseitig entsprechend der Methodenbeschreibungen in der Fachliteratur bzw. in der EN ISO 4049:2009 (Dentistry - Polymer-based restorative materials").

Im Vergleich zu dem kommerziell verfügbaren Vernetzer PETMP mit typischem Mercaptangeruch (Referenz A) bzw. methacrylatbasierenden Dentalkompositen (Referenz B) zeigte das erfindungsgemäße Kompositmaterial A deutlich geringere Schrumpfspannung und Polymerisationsschrumpfung. Die Doppelbindungsumsätze waren dabei vergleichbar mit dem PETMP-Komposit und höher als bei methacrylatbasierenden Dentalkompositen.

**Tabelle 2: Eigenschaften der Komposite**

| **Materialeigenschaften** | **Material A** | **Referenz A*)** | **Referenz B*)** |
|---|---|---|---|
| Biegefestigkeit [MPa]¹⁾ | 110 | 106 | 106 |
| Elastizitätsmodul [GPa]²⁾ | 7,7 | 7,4 | 6,3 |
| Doppelbindungsumsatz [%]³⁾ | 59 | 60 | 48 |
| Polymerisationsschrumpf [Vol.-%]⁴⁾ | 2,1 | 4,1 | 3,0 |
| Schrumpfung spannung [MPa]⁵⁾ | 4,6 | 6,8 | 6,2 |

| | | | |
|---|---|---|---|
| *) Vergleichsbeispiel ¹⁾ gemäß EN ISO 4049:2009 nach 24 h Wasserlagerung bei 37 °C ²⁾ gemäß EN ISO 4049:2009 nach 24 h Wasserlagerung bei 37 °C ³⁾ Messung mittels FTIR-ATR nach 10 min (Belichtung 20 sec, Blaulicht) ⁴⁾ Messung nach 10 min ("deflecting disc" Methode nach Watts & Cash) ⁵⁾ Messung nach 24 h (Photoelastische Methode nach Ernst | | | |

## Patentansprüche

1. Dentalwerkstoff, der mindestens ein Thiol, mindestens eine En-Verbindung mit zwei oder mehr C-C-Mehrfachbindungen und einen Photoinitiator für die radikalische Polymerisation enthält, **dadurch gekennzeichnet, dass** er mindestens ein Thiol gemäß der allgemeinen Formel (1) enthält, 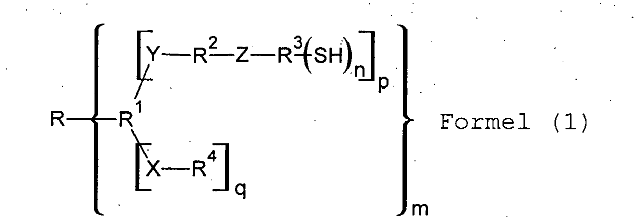 in der
R -SO₂-, ein linearer oder verzweigter aliphatischer C₁₋₂₀-Rest, ein aromatischer C₆₋₂₀-Rest, ein cycloaliphatischer C₃₋₁₈-Rest oder ein heterocyclischer Rest mit 3 bis 17 C-Atomen und 1 bis 3 Heteroatomen ist, die aus N, O und S ausgewählt sind;
R¹ entfällt oder ein linearer oder verzweigter aliphatischer C₁₋₁₂-Rest, ein aromatischer C₆₋₂₀-Rest, ein cycloaliphatischer C₃₋₁₈-Rest oder ein heterocyclischer Rest mit 3 bis 17 C-Atomen und 1 bis 3 Heteroatomen ist, die aus N, O und S ausgewählt sind;
R² entfällt oder ein linearer oder verzweigter aliphatischer C₁₋₂₀-Rest, der durch O oder S unterbrochen sein kann, ein aromatischer C₆₋₁₀-Rest, der durch CH₃, CH₂CH₃, OH, OCH₃ oder -O-CO-CH₃ substituiert sein kann;
R³ entfällt oder ein linearer oder verzweigter aliphatischer C₁₋₂₀-Rest, der durch O oder S unterbrochen sein kann, ein aromatischer C₆₋₁₀-Rest, der durch CH₃, CH₂CH₃, OH, OCH₃ oder -O-CO-CH₃ substituiert sein kann;
R⁴ ein C₁₋₆-Alkylrest ist;
X, Y unabhängig voneinander O, S, CO-NH, O-CO-NH oder NH-CO-NH sind oder entfallen;
Z O, S, CO-NH, O-CO-NH oder NH-CO-NH ist oder entfällt;
m eine ganze Zahl von 1 bis 4 ist;
n eine ganze Zahl von 1 bis 4 ist;
p eine ganze Zahl von 1 bis 6 ist;
q eine ganze Zahl von 0 bis 4 ist,
wobei n, p und m so gewählt werden, dass das Thiol insgesamt mindestens 3 SH-Gruppen aufweist, und
dass er eine Monoacyltrialkyl- oder Diacyldialkylgermanium-Verbindung als Photoinitiator enthält.

2. Dentalwerkstoff nach Anspruch 1, bei dem die Variablen der Formel 1 die folgenden Bedeutungen haben:
R -SO₂-, ein linearer oder verzweigter aliphatischer C₁₋₁₂-Rest, ein aromatischer C₆₋₁₈-Rest, ein cycloaliphatischer C₅₋₈-Rest oder ein heterocyclischer Rest mit 3 bis 5 C-Atomen, 1 bis 3 Heteroatomen und insgesamt 5-8 Ringatomen, wobei die Heteroatome aus N, O und S ausgewählt sind;
R¹ entfällt, ein linearer oder verzweigter aliphatischer C₁₋₁₀-Rest oder ein aromatischer C₆₋₁₀-Rest;
R² entfällt oder ein linearer oder verzweigter aliphatischer C₁₋₁₀-Rest oder ein Phenylrest;
R³ ein linearer oder verzweigter aliphatischer C₁₋₁₀-Rest oder ein Phenylrest;
R⁴ ein C₁₋₄-Alkylrest;
X O oder entfällt;
Y O oder entfällt;
Z O oder entfällt;
m 1, 2 oder 3;
n 1 oder 2;
p 1, 2 oder 3;
q 0, 1 oder 2.

3. Dentalwerkstoff nach Anspruch 1 oder 2, bei dem die Variablen der Formel 1 die folgenden Bedeutungen haben:
R -SO₂-, ein linearer oder verzweigter aliphatischer C₁₋₆-Rest, ein aromatischer C₆-Rest, ein cycloaliphatischer C₅₋₈-Rest oder ein 1,3,5-Triazin-2,4,6-trionrest;
R¹ entfällt, ein linearer oder verzweigter aliphatischer C₁₋₆-Rest oder ein Phenylrest;
R² entfällt oder ein linearer oder verzweigter aliphatischer C₁₋₃-Rest;
R³ ein linearer oder verzweigter aliphatischer C₂₋₆-Rest;
R⁴ ein C₁₋₃-Alkylrest;
X O oder entfällt;
Y O oder entfällt;
Z O oder entfällt;
m 2 oder 3;
n 1;
p 1 oder 2;
q 0 oder 1.

4. Dentalwerkstoff nach einem der Ansprüche 1 bis 3, in dem das Thiol der allgemeinen Formel (1) als Reaktionsprodukt mit einem di- oder multifunktionellen Acrylat oder Acrylamid oder mit einem di- oder multifunktionellen Isocyanat vorliegt.

5. Dentalwerkstoff nach einem der Ansprüche 1 bis 4, der als En-Verbindung eine Vinyl-, Allyl- oder Norbornenverbindung oder ein Alkin enthält.

6. Dentalwerkstoff nach Anspruch 5, der als En-Verbindung
einen Vinylether, Vinylester oder ein N-Vinylamid;
und/oder
einen Allylether von tri- oder höherfunktionalisierten Alkoholen oder ein Umsetzungsprodukt von tri- oder höherfunktionalisierten Carbonsäuren mit Allylalkohol oder Allylamin, ein Triallylamin oder Triallyl-1,3,5-triazin-2,4,6-trion (TATATO);
und/oder
einen Ester von 5-Norbornen-2-carbonsäure mit tri- oder höherfunktionalisierten Alkoholen oder einen Ester von 5-Norbornen-2-methanol mit tri- oder höherfunktionalisierten Carbonsäuren;
und/oder
einen Ester von Propargylalkohol mit tri- oder höherfunktionalisierten Carbonsäuren oder einen Ether von Propargylalkohol mit tri- oder höherfunktionalisierten Alkoholen enthält.

7. Dentalwerkstoff nach einem der Ansprüche 1 bis 6, der zusätzlich mindestens ein mono- oder multifunktionelles Methacrylat oder eine Mischung davon enthält.

8. Dentalwerkstoff nach einem der Ansprüche 1 bis 7, der als Photoinitiator Benzoyltrimethylgermanium, Dibenzoyldiethylgermanium oder Bis (4-methoxybenzoyl)diethyl-germanium enthält.

9. Dentalwerkstoff nach einem der Ansprüche 1 bis 8, der zusätzlich organischen oder anorganischen partikulären Füllstoff enthält.

10. Dentalwerkstoff nach einem der Ansprüche 1 bis 9, der die folgenden Komponenten enthält:
a) 5 bis 40 Gew.-%, bevorzugt 5 bis 30 Gew.-% und besonders bevorzugt 5 bis 20 Gew.-% mindestens eines Thiols der allgemeinen Formel I oder eines Oligomers davon,
b) 5 bis 40 Gew.-%, bevorzugt 5 bis 30 Gew.-% und besonders bevorzugt 5 bis 20 Gew.-% mindestens einer En-Komponente,
c) 0 bis 40 Gew.-%, bevorzugt 2 bis 30 Gew.-% und besonders bevorzugt 4 bis 20 Gew.-% Methacrylat(e),
d) 0,01 bis 10 Gew.-%, besonders bevorzugt 0,1 bis 3,0 Gew.-% an Initiator(en),
e) 10 bis 85 Gew.-% Füllstoff(e), und
f) 0 bis 10 Gew.-%, bevorzugt 0 bis 5 Gew.-% Additiv(e).

11. Dentalwerkstoff nach einem der Ansprüche 1 bis 10 zur intraoralen Anwendung als dentaler Zement, Füllungskomposit oder Verblendmaterial.

12. Verwendung eines Dentalwerkstoffs gemäß einem der Ansprüche 1 bis 10 zur Herstellung von eines Inlays, Onlays, Krone oder Brücke.

13. Verfahren zur Herstellung eines oligomeren Thiols, bei dem man ein Thiol der Formel (I) gemäß Anspruch 1 im stöchiometrischen Überschuss mit einer di- oder multifunktionellen En-Verbindung oder mit einem di- oder multifunktionellen Isocyanat umsetzt.

14. Verfahren nach Anspruch 13, bei dem man das Thiol der Formel (I) mit einem di- oder multifunktionellen Acrylat, di-oder multifunktionellen Acrylamid oder di- oder multifunktionellen Isocyanat in einem molaren Verhältnis von SH- zu Acryl- bzw. NCO-Gruppen von 1,5 bis 15 : 1, vorzugsweise 1,5 bis 9 : 1 umsetzt.

15. Oligomeres Thiol, das nach einem Verfahren gemäß Anspruch 13 oder 14 erhältlich ist.

## Claims

1. Dental material, which comprises at least one thiol, at least one ene compound with two or more C-C multiple bonds, and a photoinitiator for radical polymerisation, **characterised in that** said dental material comprises at least one thiol according to the general Formula (1), 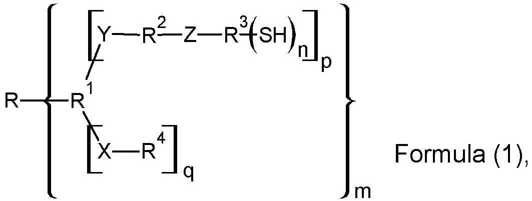 in which
R is -SO₂-, a linear or branched aliphatic C₁₋₂₀ group, an aromatic C₆₋₂₀ group, a cycloaliphatic C₃₋₁₈ group or a heterocyclic group with 3 to 17 C atoms and 1 to 3 heteroatoms that are selected from N, O and S;
R¹ is absent or is a linear or branched aliphatic C₁₋₁₂ group, an aromatic C₆₋₂₀ group, a cycloaliphatic C₃₋₁₈ group or a heterocyclic group with 3 to 17 C atoms and 1 to 3 heteroatoms that are selected from N, O and S;
R² is absent or is a linear or branched aliphatic C₁₋₂₀ group that may be interrupted by O or S, an aromatic C₆₋₁₀ group that may be substituted by CH₃, CH₂CH₃, OH, OCH₃ or -O-CO-CH₃;
R³ is absent or is a linear or branched aliphatic C₁₋₂₀ group that may be interrupted by O or S, an aromatic C₆₋₁₀ group that may be substituted by CH₃, CH₂CH₃, OH, OCH₃ or -O-CO-CH₃;
R⁴ is a C₁₋₆ alkyl group;
X, Y independently of each other are O, S, CO-NH, O-CO-NH or NH-CO-NH or are absent;
Z is O, S, CO-NH, O-CO-NH or NH-CO-NH or is absent;
m is an integer from 1 to 4;
n is an integer from 1 to 4;
p is an integer from 1 to 6;
q is an integer from 0 to 4,
wherein n, p and m are chosen such that the thiol has a total of at least 3 SH groups, and
that said dental material comprises a monoacyltrialkylgermanium compound or a diacyldialkylgermanium compound as the photoinitiator.

2. Dental material according to claim 1, in which the variables of Formula 1 have the following meanings:
R is -SO₂-, a linear or branched aliphatic C₁₋₁₂ group, an aromatic C₆₋₁₈ group, a cycloaliphatic C₅₋₈ group or a heterocyclic group with 3 to 5 C atoms, 1 to 3 heteroatoms and a total of 5-8 ring atoms, wherein the heteroatoms are selected from N, O and S;
R¹ is absent, a linear or branched aliphatic C₁₋₁₀ group or an aromatic C₆₋₁₀ group;
R² is absent or is a linear or branched aliphatic C₁₋₁₀ group or a phenyl group;
R³ is a linear or branched aliphatic C₁₋₁₀ group or a phenyl group;
R⁴ is a C₁₋₄ alkyl group;
X is O or is absent;
Y is O or is absent;
Z is O or is absent;
m is 1, 2 or 3;
n is 1 or 2;
p is 1, 2 or 3;
q is 0, 1 or 2.

3. Dental material according to claim 1 or 2, in which the variables of Formula 1 have the following meanings:
R is -SO₂-, a linear or branched aliphatic C₁₋₆ group, an aromatic C₆ group, a cycloaliphatic C₅₋₈ group or a 1,3,5-triazine-2,4,6-trione group;
R¹ is absent, a linear or branched aliphatic C₁₋₆ group or a phenyl group;
R² is absent or is a linear or branched aliphatic C₁₋₃ group;
R³ is a linear or branched aliphatic C₂₋₆ group;
R⁴ is a C₁₋₃ alkyl group;
X is O or is absent;
Y is O or is absent;
Z is O or is absent;
m is 2 or 3;
n is 1;
p is 1 or 2;
q is 0 or 1.

4. Dental material according to one of claims 1 to 3, in which the thiol of general Formula (1) is present as the reaction product with a di- or multi-functional acrylate or acrylamide or with a di- or multi-functional isocyanate.

5. Dental material according to one of claims 1 to 4 which comprises a vinyl, allyl or norbornene compound or an alkyne as the ene compound.

6. Dental material according to claim 5 which comprises as the ene compound
a vinyl ether, vinyl ester or an *N*-vinylamide;
and/or
an allyl ether of tri- or higher-functionalised alcohols or a reaction product of tri- or higher-functionalised carboxylic acids with allyl alcohol or allylamine, a triallylamine or triallyl-1,3,5-triazine-2,4,6-trione (TATATO);
and/or
an ester of 5-norbornene-2-carboxylic acid with tri- or higher-functionalised alcohols or an ester of 5-norbornene-2-methanol with tri- or higher-functionalised carboxylic acids;
and/or
an ester of propargyl alcohol with tri- or higher-functionalised carboxylic acids or an ether of propargyl alcohol with tri- or higher-functionalised alcohols.

7. Dental material according to one of claims 1 to 6 which additionally comprises at least one mono- or multi-functional methacrylate or a mixture thereof.

8. Dental material according to one of claims 1 to 7 which comprises benzoyltrimethylgermanium, dibenzoyldiethylgermanium or *bis*(4-methoxybenzoyl)diethylgermanium as the photoinitiator.

9. Dental material according to one of claims 1 to 8 which additionally comprises an organic or inorganic particulate filler.

10. Dental material according to one of claims 1 to 9 which comprises the following components:
a) 5 to 40 wt %, preferably 5 to 30 wt % and particularly preferably 5 to 20 wt % of at least one thiol of the general Formula 1 or an oligomer thereof,
b) 5 to 40 wt %, preferably 5 to 30 wt % and particularly preferably 5 to 20 wt % of at least one ene component,
c) 0 to 40 wt %, preferably 2 to 30 wt % and particularly preferably 4 to 20 wt % of methacrylate(s),
d) 0.01 to 10 wt %, particularly preferably 0.1 to 3.0 wt % of initiator(s),
e) 10 to 85 wt % of filler(s), and
f) 0 to 10 wt %, preferably 0 to 5 wt % of additive(s).

11. Dental material according to one of claims 1 to 10 for intraoral use as dental cement, filling composite or veneering material.

12. Use of a dental material according to one of claims 1 to 10 for preparing an inlay, onlay, crown or bridge.

13. Process for preparing an oligomeric thiol in which a stoichiometric excess of a thiol of Formula (1) according to claim 1 is reacted with a di- or multi-functional ene compound or with a di- or multi-functional isocyanate.

14. Process according to claim 13 in which the thiol of Formula (1) is reacted with a di- or multi-functional acrylate, di- or multi-functional acrylamide or di- or multi-functional isocyanate in a molar ratio of SH to acrylic or NCO groups of from 1.5:1 to 15:1, preferably 1.5:1 to 9:1.

15. Oligomeric thiol that is obtainable using a process according to claim 13 or 14.

## Revendications

1. Matériau dentaire, qui contient au moins un thiol, au moins un composé ène comportant deux ou plus de deux liaisons C-C multiples et un photoamorceur pour la polymérisation radicalaire, **caractérisé en ce qu'**il contient au moins un thiol selon la formule générale (1), 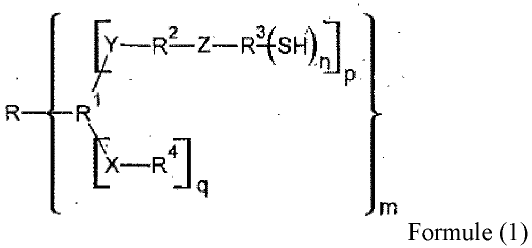 dans laquelle
R est un groupe -SO₂-, un radical aliphatique en C₁-C₂₀ linéaire ou ramifié, un radical aromatique en C₆-C₂₀, un radical cycloaliphatique en C₃-C₁₈ ou un radical hétérocyclique ayant de 3 à 17 atomes de carbone et de 1 à 3 hétéroatomes qui sont choisis parmi les atomes d'azote, d'oxygène et de soufre ;
R¹ est absent ou représente un radical aliphatique en C₁-C₁₂ linéaire ou ramifié, un radical aromatique en C₆-C₂₀, un radical cycloaliphatique en C₃-C₁₈ ou un radical hétérocyclique ayant de 3 à 17 atomes de carbone et de 1 à 3 hétéroatomes qui sont choisis parmi les atomes d'azote, d'oxygène et de soufre ;
R² est absent ou représente un radical aliphatique en C₁-C₂₀ linéaire ou ramifié qui peut être interrompu par O ou S, un radical aromatique en C₆-C₁₀ qui peut être substitué par CH₃, CH₂CH₃, OH, OCH₃ ou -O-CO-CH₃ ;
R³ est absent ou représente un radical aliphatique en C₁-C₂₀ linéaire ou ramifié qui peut être interrompu par O ou S, un radical aromatique en C₆-C₁₀ qui peut être substitué par CH₃, CH₂CH₃, OH, OCH₃ ou -O-CO-CH₃ ;
R⁴ est un radical alkyle en C₁-C₆ ;
X, Y indépendamment l'un de l'autre représentent un atome d'oxygène ou de soufre ou un groupe CO-NH, O-CO-NH ou NH-CO-NH ou sont absents ;
Z est un atome d'oxygène ou de soufre ou un groupe CO-NH, O-CO-NH ou NH-CO-NH ou est absent ;
m est un nombre entier valant de 1 à 4 ;
n est un nombre entier valant de 1 à 4 ;
p est un nombre entier valant de 1 à 6 ;
q est un nombre entier valant de 0 à 4,
n, p, et m étant choisis de manière que le thiol comporte au total au moins 3 groupes SH, et
**en ce qu'**il contient un composé monoacyltrialkyl- ou diacyldialkylgermanium en tant que photoamorceur.

2. Matériau dentaire selon la revendication 1, dans lequel les variables de la formule 1 ont les significations suivantes :
R est un groupe -SO₂-, un radical aliphatique en C₁-C₁₂ linéaire ou ramifié, un radical aromatique en C₆-C₁₈, un radical cycloaliphatique en C₅-C₈ ou un radical hétérocyclique ayant de 3 à 5 atomes de carbone, de 1 à 3 hétéroatomes et au total 5-8 atomes formant le cycle, les hétéroatomes étant choisis parmi les atomes d'azote, d'oxygène et de soufre ;
R¹ est absent ou représente un radical aliphatique en C₁-C₁₀ linéaire ou ramifié, ou un radical aromatique en C₆-C₁₀ ;
R² est absent ou représente un radical aliphatique en C₁-C₁₀ linéaire ou ramifié ou un radical phényle ;
R³ est un radical aliphatique en C₁-C₁₀ linéaire ou ramifié ou un radical phényle ;
R⁴ est un radical alkyle en C₁-C₄ ;
X représente un atome d'oxygène ou est absent ;
Y représente un atome d'oxygène ou est absent ;
Z représente un atome d'oxygène ou est absent ;
m vaut 1, 2 ou 3 ;
n vaut 1 ou 2 ;
p vaut 1, 2 ou 3 ;
q vaut 0, 1 ou 2.

3. Matériau dentaire selon la revendication 1 ou 2, dans lequel les variables de la formule 1 ont les significations suivantes :
R est un groupe -SO₂-, un radical aliphatique en C₁-C₆ linéaire ou ramifié, un radical aromatique en C₆, un radical cycloaliphatique en C₅-C₈ ou un radical 1,3,5-triazine-2,4,6-trione ;
R¹ est absent ou représente un radical aliphatique en C₁-C₆ linéaire ou ramifié, ou un radical phényle ;
R² est absent ou représente un radical aliphatique en C₁-C₃ linéaire ou ramifié ;
R³ est un radical aliphatique en C₂-C₆ linéaire ou ramifié ;
R⁴ est un radical alkyle en C₁-C₃ ;
X représente un atome d'oxygène ou est absent ;
Y représente un atome d'oxygène ou est absent ;
Z représente un atome d'oxygène ou est absent ;
m vaut 2 ou 3 ;
n vaut 1 ;
p vaut 1 ou 2 ;
q vaut 0 ou 1.

4. Matériau dentaire selon l'une quelconque des revendications 1 à 3, dans lequel le thiol de formule générale (1) est présent sous forme de produit de réaction avec un acrylate ou acrylamide di- ou multifonctionnel ou avec un isocyanate di- ou multifonctionnel.

5. Matériau dentaire selon l'une quelconque des revendications 1 à 4, qui contient en tant que composé ène un composé vinylique, allylique ou norbomène ou un alcyne.

6. Matériau dentaire selon la revendication 5, qui contient en tant que composé ène
un éther vinylique, ester vinylique ou un N-vinylamide ;
et/ou
un éther allylique d'alcools trifonctionnalisés ou à plus haut degré de fonctionnalisation ou un produit de réaction d'acides carboxyliques trifonctionnalisés ou à plus haut degré de fonctionnalisation avec l'alcool allylique ou l'allylamine, une triallylamine ou la triallyl-1,3,5-triazine-2,4,5-trione (TATATO) ;
et/ou
un ester d'acide 5-norbonène-2-carboxylique avec des alcools trifonctionnalisés ou à plus haut degré de fonctionnalisation ou un ester de 5-norbornène-2-méthanol avec des acides carboxyliques trifonctionnalisés ou à plus haut degré de fonctionnalisation ;
et/ou
un ester d'alcool propargylique avec des acides carboxyliques trifonctionnalisés ou à plus haut degré de fonctionnalisation ou un éther d'alcool propargylique avec des alcools trifonctionnalisés ou à plus haut degré de fonctionnalisation.

7. Matériau dentaire selon l'une quelconque des revendications 1 à 6, qui contient en outre au moins un méthacrylate mono- ou multifonctionnel ou un mélange de tels méthacrylates.

8. Matériau dentaire selon l'une quelconque des revendications 1 à 7, qui contient en tant que photoamorceur du benzoyltriméthylgermanium, du dibenzoyl-diéthylgermanium ou du bis(4-méthoxybenzoyl)diéthylgermanium.

9. Matériau dentaire selon l'une quelconque des revendications 1 à 8, qui contient en outre des charges particulaires organiques ou inorganiques.

10. Matériau dentaire selon l'une quelconque des revendications 1 à 9, qui contient les composants suivants :
a) 5 à 40 % en poids, de préférence 5 à 30 % en poids et de façon particulièrement préférée 5 à 20 % en poids d'au moins un thiol de formule générale I ou un oligomère de celui-ci,
b) 5 à 40 % en poids, de préférence 5 à 30 % en poids et de façon particulièrement préférée 5 à 20 % en poids d'au moins un composant ène,
c) 0 à 40 % en poids, de préférence 2 à 30 % en poids et de façon particulièrement préférée 4 à 20 % en poids de méthacrylate(s),
d) 0,01 à 10 % en poids, de façon particulièrement préférée 0,1 à 3,0 % en poids d'amorceur(s),
e) 10 à 85 % en poids de charge(s), et
f) 0 à 10 % en poids, de préférence 0 à 5 % en poids d'additif(s).

11. Matériau dentaire selon l'une quelconque des revendications 1 à 10, destiné à l'utilisation intra-orale en tant que ciment dentaire, composite d'obturation ou matériau de placage.

12. Utilisation d'un matériau dentaire selon l'une quelconque des revendications 1 à 10 pour la production d'un inlay, d'un onlay, d'une couronne ou d'un bridge.

13. Procédé pour la préparation d'un thiol oligomère, dans lequel on fait réagir un thiol de formule (I) selon la revendication 1 en excès stoechiométrique avec un composé ène di- ou multifonctionnel ou avec un isocyanate di- ou multifonctionnel.

14. Procédé selon la revendication 13, dans lequel on fait réagir le thiol de formule (I) avec un acrylate di- ou multifonctionnel, acrylamide di- ou multifonctionnel ou isocyanate di- ou multifonctionnel en un rapport molaire des groupes SH aux groupes acryloyle ou NCO de 1,5 à 15 : 1, de préférence de 1,5 à 9 : 1.

15. Thiol oligomère, qui peut être obtenu conformément à un procédé selon la revendication 13 ou 14.
